(19) Europäisches Patentamt / European Patent Office / Office européen des brevets

(11) **EP 3 646 030 B1**

(12) **EUROPEAN PATENT SPECIFICATION**


(45) Date of publication and mention of the grant of the patent:
**09.11.2022 Bulletin 2022/45**

(21) Application number: **18747010.9**

(22) Date of filing: **27.06.2018**

(51) International Patent Classification (IPC):
***G01N 33/543*** *(2006.01)*

(52) Cooperative Patent Classification (CPC):
**G01N 33/54373**

(86) International application number:
**PCT/IB2018/054763**

(87) International publication number:
**WO 2019/003144 (03.01.2019 Gazette 2019/01)**


(54) **ULTRASENSITIVE CANTILEVER**

ULTRASENSITIVER AUSLEGER

CANTILEVER ULTRASENSIBLE


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2017 PCT/IB2017/053956**

(43) Date of publication of application:
**06.05.2020 Bulletin 2020/19**



(73) Proprietor: **3P SENSE LIMITED**
**2nd Floor**
**London W1D 5EU (GB)**

(72) Inventor: **WAFULA NDIEYIRA, Joseph**
**London NW3 2TX (GB)**

(74) Representative: **Biggi, Cristina**
**Bugnion S.p.A.**
**Viale Lancetti 17**
**20158 Milano (IT)**



(56) References cited:
**WO-A1-2009/143373**
**US-A1- 2003 068 446**
**US-A1- 2006 075 803**

- **FRITZ J ET AL: "TRANSLATING BIOMOLECULAR RECOGNITION INTO NANOMECHANICS", SCIENCE, AMERICAN ASSOCIATION FOR THE ADVANCEMENT OF SCIENCE, vol. 288, 14 April 2000 (2000-04-14), pages 316-319, XP000971747, ISSN: 0036-8075, DOI: 10.1126/SCIENCE.288.5464.316**
- **LEE K-B ET AL: "PROTEIN NANOARRAYS GENERATED BY DIP-PEN NANOLITHOGRAPHY", SCI, AMERICAN ASSOCIATION FOR THE ADVANCEMENT OF SCIENCE, vol. 295, 7 February 2002 (2002-02-07), pages 1702-1705, XP008000827, ISSN: 0036-8075, DOI: 10.1126/SCIENCE.1067172**
- **JOSEPH W. NDIEYIRA ET AL: "Surface-stress sensors for rapid and ultrasensitive detection of active free drugs in human serum", NATURE NANOTECHNOLOGY, vol. 9, no. 3, 2 March 2014 (2014-03-02), pages 225-232, XP055405272, GB ISSN: 1748-3387, DOI: 10.1038/nnano.2014.33**
- **NDIEYIRA JOSEPH W ET AL: "Surface mediated cooperative interactions of drugs enhance mechanical forces for antibiotic action", SCIENTIFIC REPORTS,, vol. 7, 23 February 2017 (2017-02-23), pages 41206-1, XP009195443, ISSN: 2045-2322, DOI: 10.1038/SREP41206**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 3 646 030 B1

## Description

### Technical field

The present invention relates to cantilever sensors and cantilever sensor arrays having highly reproducible and ultrasensitive signal response for the detection of molecules in body fluids.

### Background art

There has been a growing appreciation in recent years that quantitative analysis of mechanical signals generated from molecular interactions could be as useful as the chemical and electrical signaling generated in molecular assays. The ability to probe molecular interactions to produce biologically relevant, quantifiable and reproducible signals will shed light on a wide range of complicated tasks performed by living cells in both health and disease, and will clarify a number of clinical disorders. For example, in many biological systems[1-3] the remarkable ability of molecules to associate into complex structures and the underlying biomechanical forces[4-7] generated determine the signal response from different cascades of functional activity. The biomechanical forces arising from molecular interactions between cells and their microenvironment and the signals generated also play a vital role in embryonic development and adult physiology as well as in disease states such as cancer among many others[8,9]. Cantilever sensing platform based on microfabricated arrays of silicon[10-12] represent a versatile operation for biodetection. This could potentially be exploited to probe molecular interactions and quantify the associated biomechanical forces with high sensitivity, specificity and reproducibility to enable a better understanding of biological systems and provide a new basis for developing precise diagnostic tools in areas where stringent data consistency is vital such as in healthcare, food safety and environment among others. The binding force between a receptor and its target in solution is mechanically transduced to a cantilever resulting into a resonance frequency shift[13-17] or deflection[18-20] which is directly proportional to the ligand concentration. The inherent sensitivity of mechanical devices of microscale dimensions to miniscule forces has been exploited for the analysis of a wide range of biologically relevant targets including studying the binding kinetics and nanometrology of antibiotics[4,11,13,15,21,22], visualization of charge flow in proteins[23], biological imaging for nanoscale characterization of plant cell walls[24] and microbial cell surfaces[25,26], as well as genotyping of cancer cells[27]. While this technology has evolved beyond the well-established realm of imaging to innovative applications in biomedical analysis, the variability in signal response obtained even when measurements are conducted under rigorous conditions, remains a significant challenge.

Reliable and reproducible signal response is critical for standardization of data particularly if assays are to be transferred between laboratories. Stringent data consistency is also vital before commercial cantilever biosensors can expand their reach into vital areas such as healthcare, the environment and the food industry where data reproducibility is paramount.

Different approaches have been implemented in an attempt to address this problem. The issues arising from human error, environmental or technical fluctuations have easily been resolved by automation.

Other strategies include altering morphology of cantilever surfaces[10], receptor grafting density[22], receptor orientation[28] or passivation of the silicon surface[22,29]. Remarkably the science behind these large variations in signal response has remained unresolved for more than 20 years. Therefore, the creation of a reliable nanomechanical assays, would benefit from a clearer understanding of the specific sequences of events of localized biomechanical forces on a receptor molecule, redistribution through cognate network of transduction arrays and induction of global biomechanical force, which directly determines the signal response at which the problem of variability could be addressed.

The inventor of the present disclosure has: (1) demonstrated for the first time that mechanical continuity between surface receptor networks can be reprogrammed to deliver highly reproducible signals with significantly improved detection sensitivity; (2) demonstrated that mechanical connectivity networks between surface receptors, the plurality of the global force networks and a hinge region, defined as the anchoring area between one end of the sensing element and a preclamped solid support, affect the net biomechanical force which determines signal sensitivity and reproducibility; (3) demonstrated that the mechanical connectivity both in terms of longitudinal and transverse formation is increased when the geometrical width of the region covered by surface receptors is increased; (4) demonstrated for the first time that chiral species of a drug molecule and its target generate distinct biomechanical forces, which can be accurately and reproducibly measured. The experiments conducted by the inventor of the present disclosure (here reported) demonstrate that there are three essential criteria which must work cooperatively in order to provide the basis of reprogrammable and reproducible signaling. First, there must be continuous connectivity between the hinge region and cognate network of receptors along the length of the sensing element. Second, mechanical connectivity must be over an appreciable length of receptor patching. Third, there must be a creation of a relatively large fraction of surface coverage.

In addition, the inventor found that reducing the width of the cantilever below the conventional 100 $\mu$m, preferably to a width of equal or less than 70 $\mu$m, dramatically improves the limits of detection down to sub-femtomolar quantities, without compromising the signal reproducibility or the need to use sample labelling.

## Summary of the invention

The present invention relates to a cantilever sensor having two sides, one of which is coated with a gold layer. The cantilever sensor is included in an array comprising a plurality of cantilevers, each one anchored at only one end to a support from which they protrude. The anchoring area between one end of the cantilever and a support is called hinge region.

The gold layer has a surface that is functionalized with a self-assembled monolayer (SAM) of a probe molecule. The self-assembled monolayer (SAM) of a probe molecule is composed of a plurality of probe molecules each one connected to the surface of the gold layer through a linker. The self-assembled monolayer (SAM) of a probe molecule can also be defined as a network of probe molecules or SAM network.

The plurality of probe molecules that compose the SAM covers between 30% and 80% of the gold layer surface and is arranged along the longitudinal length of the cantilever or transverse to the long axis of the cantilever in a continuous connectivity between the hinge region and the cognate network of probe molecules. The continuous connectivity is present also among the plurality of probe molecules composing the network (or SAM network) of probe molecules. The continuous connectivity is achieved if the distance (i.e. the length) between the hinge region as well as the network of probe molecules and among the plurality of a probe molecule arranged longitudinally along the length of the cantilever is equal or less than 50 $\mu$m. Such continuous mechanical networks result in the intensification of both short and long-range interactions leading to an enhanced longitudinal force and mechanical response.

The inventor has demonstrated that if the distance between the hinge region and the network of probe molecules is greater than 50 $\mu$m then the cantilever bending response is about zero or very small because the mechanical connectivity of the network of probe molecules and the hinge region is decoupled, which leads to the global longitudinal force to be significantly diminished to the extent that it is insufficient to propagate a mechanical response. Subsequently, if the separation distance to the hinge region is > 50 $\mu$m, the reproducibility of the signal response is negatively influenced or even totally disrupted.

In a preferred embodiment, the cantilever has a width that is $\leq$ 100 $\mu$m, preferably $\leq$ 70 $\mu$m. The inventor has demonstrated that a narrow cantilever improves detection sensitivity.

The cantilever can be passivated on the side that is not coated with the SAM with, for example, a PEG-silane coating. Alternatively, the cantilever is not passivated on one side. The invention relates also to a method for detecting the presence of a coupling molecule in an isolated body fluid using the cantilever. In particular, the cantilever of the invention enables detection of distinct mechanical signaling generated by biologically relevant molecules and that this can be accurately and reproducibly measured. This has important implications for the rapid discrimination between individual drug enantiomers and also for the future design of more effective drugs to control infections as well as for determining dose levels.

## Brief description of the drawings

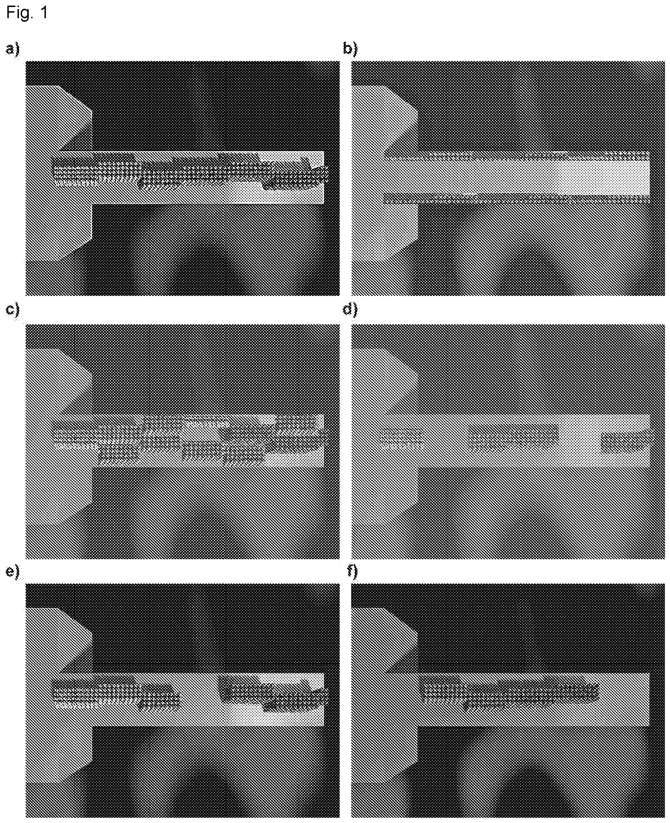

Figures 1 a-f show the impact of mechanical connectivity on local and global signal response; a, Schematic representation of array networks (cartoons) arranged centrally and overlapping longitudinally along the entire length of a cantilever and continuous with the hinge region. b, Array networks arranged longitudinally at the edges of a cantilever and continuous with the hinge region. c, Array networks arranged randomly in which arrays overlap with the adjacent array to create a continuous connectivity with one another and with the hinge region. d, Array networks arranged randomly but without continuity with each other or the hinge region. e, Array networks of mechanical connectivity arranged longitudinally and continuously with the hinge region and the free-end of the cantilever but decoupled at the centre. f, Array networks of continuous mechanical connectivity arranged longitudinally but confined to the centre of a cantilever only. In a-c, an excellent force response was obtained as a result of continuous mechanical connectivity between the networks and hinge region. In d, a negligible force response was detected as a result of discontinuous mechanical connectivity. In e, the measured force was diminished as a result of discontinuous connectivity at the centre of the mechanical networks. In f, no force was detected due to the absence of connectivity between the mechanical networks and hinge region. In a-f, the un-patterned areas on the Au-cantilever surface (without cartoons) were passivated to block nonspecific interactions. The configurations demonstrate the impact of connectivity between the mechanical array networks and hinge region on signal transduction.

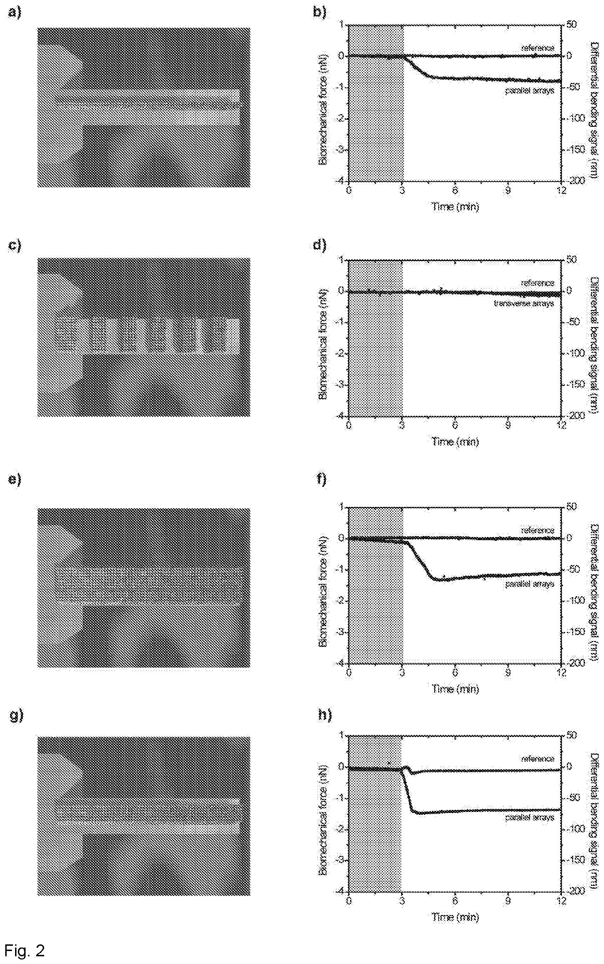

Figures 2 a-h show the results of examining a hinge region and its impact on signal reproducibility, in particular they show a, Schematic representation of self-assembled monolayers (SAMs) of mercaptoundecanoic acid (MUA) array networks arranged as a narrow strip (30% of the total surface area) running centrally along the entire length of a cantilever and continuous with the hinge region. b, Corresponding signal response obtained when the pH of the surrounding solution was switched from 4.8 to 9.0 to create a compressive biomechanical force on the Au surface. c, Array networks of MUA SAMs arranged in strips transverse to the long axis of the cantilever creating mechanical networks which are discontinuous with each other and with the hinge region. d, The resulting force response when the pH of the surrounding solution was increased from 4.8 to 9.0. e, MUA SAMs covering the entire surface of the cantilever surface creating a mechanical network which is continuous with the hinge region. f, Corresponding force response when the pH of the surrounding solution was changed from 4.8 to 9.0. g, Array networks of SAMs of mercaptohexadecanoic acid (MHA) array networks arranged as a broad strip (50% of the total surface area) running centrally along the entire length of a cantilever and continuous with the hinge region. In a, c and g, the un-patterned areas on the cantilever were passivated using SAMs of undecanethiol (UDT) in the case of MUA, and hexadecanethiol (HDT) for MHA to block nonspecific interactions. h, Corresponding biomechanical force response of MHA array networks obtained when the pH of the surrounding solution was switched from 4.8 to 9.0 to create a compressive biomechanical force on the Au surface. In b, d, f and h, the shaded areas represent the 3 min time frame during which sodium phosphate buffer (pH 4.8) was injected to establish a baseline. Downward bending of the cantilever corresponds to compressive biomechanical force on the Au surface and the results demonstrate the impact that continuity of mechanical connectivity with the hinge region has on the signal response.

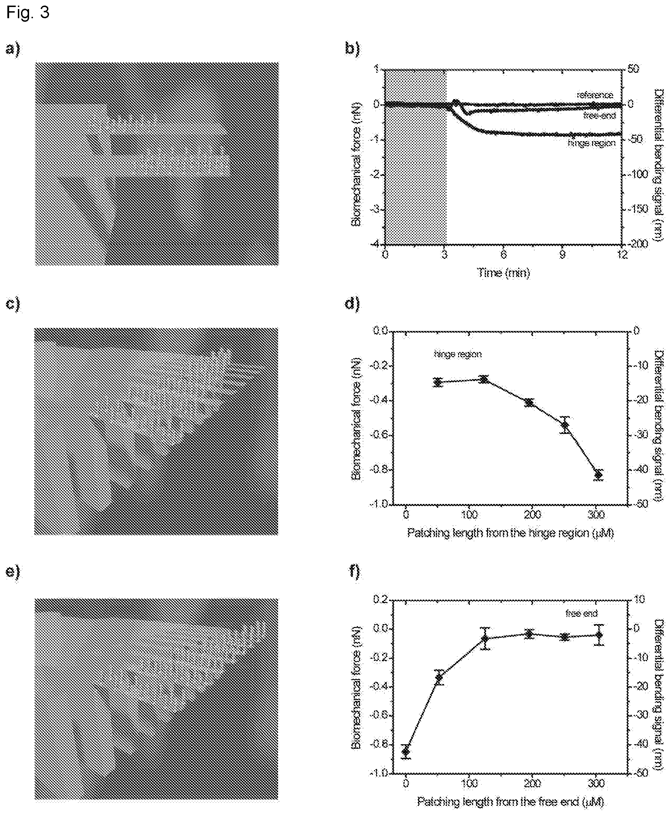

Figures 3 a-f show the results of tests relating to the decoupling of continuous mechanical networks from the hinge region to re-programme a reproducible and amplified signal response; a, First, array networks of MUA SAMs (cartoons) arranged continuously from the hinge region and terminating at the centre of the cantilever. Second, array networks of MUA SAMs arranged continuously from the free end of the cantilever and terminating at the centre of the cantilever. b, The corresponding mechanical force response of array networks starting from free-end (small signal response) or hinge region (large signal response) obtained when the pH of the surrounding solution was switched from 4.8 to 9.0 to create a compressive biomechanical force on the Au surface. The shaded areas represent the 3 min time frame during which sodium phosphate buffer (pH 4.8) was injected to establish a baseline. Downward bending of the cantilever corresponds to compressive biomechanical force on the Au surface. c, Array networks of MUA SAMs arranged continuously from the hinge region and terminating at varying distances from the free end of the cantilever. d, Corresponding biomechanical force response plotted as a function of length of cantilever-receptor coverage. e, Array networks of MUA SAMs arranged continuously from the free end of the cantilever and terminating at varying distances from the hinge region. In a, c and e, the un-patterned areas on the Au-cantilever surface (without cartoons) were passivated using SAMs of undecanethiol (UDT) to block nonspecific interactions. f, Corresponding force response plotted as a function of length of cantilever-receptor coverage. In d and f, the solid lines connecting the diamond and circle data points are for visual guidance only and not fitted to any particular equation. In e and f, the error bars represent the standard deviation of the force. The results demonstrate that continuity with the hinge region with a minimum critical distance of around -50 um is crucial for generating a consistent signal response.

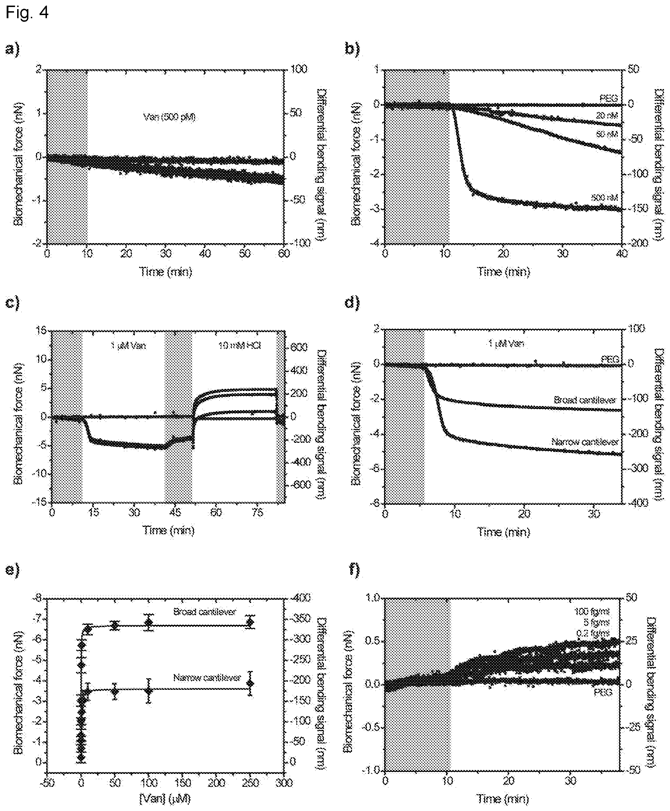

Figures 4a-f show the influence of sensor geometry and mechanical connectivity on signal response; a, Mechanical forces arising from 500 pM vancomycin (Van) in sodium phosphate buffer solution at pH 7.4 against vancomycin susceptible receptor (VSR) coated on a small sized cantilever sensor. The signal mechanical response of ~-400 $\pm$ 30 pN represent data obtained from three separate cantilever experiments. b, Biomechanical force response when the same experiment was repeated with 20 nM (~-500 pN), 50 nM ((~-1500 pN) and 500 nM ((~-3000 pN) Van using one cantilever. c, Mechanical force response with Van at 1 $\mu$M concentration using 3 separate cantilevers (--5000 $\pm$ 400 pN). d, Mechanical force response obtained with 1 $\mu$M Van versus VSR coated on a small sized cantilever (blue) and large sized cantilever (red). In a - d, downward bending of the cantilever corresponds to compressive biomechanical force on the Au surface. e, Plot of mechanical forces arising from [Van] against VSR using small sized cantilever (--6900 pN) and large sized cantilever (--3800 pN). The data obtained, represented by diamonds and circles, was used to calculate Kd using equation (1) and the error bars shown represent the standard deviation of the signal response. f, Mechanical forces obtained from 0.2 fg ml-1 (-200 pN), 5 fg ml-1 (-350 pN) and 100 fg ml-1 (-500 pN) immunoglobulin G (IgG) in sodium phosphate buffer solution pH 7.4 against anti-mouse IgG coated 70 $\mu$m wide cantilevers. An upward cantilever bending corresponds to a tensile biomechanical force. In a - d and f the shaded areas represent the time frame during which sodium phosphate buffer (pH 7.4) without a ligand is injected into the liquid cell for 5 or 10 min in order to establish a baseline. The control signal from polyethylene glycol (PEG) coated cantilevers is shown in black. The results indicate that mechanical connectivity of receptors to a hinge region plays a significant role in signal reproducibility and is independent of the geometry of the sensing element.

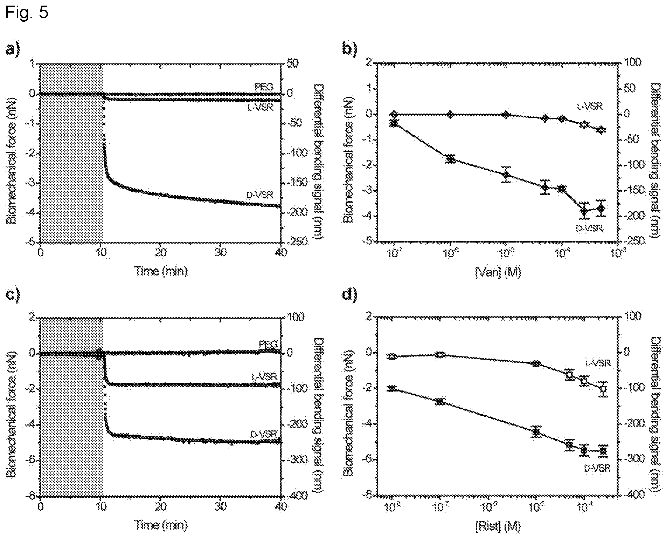

Figures 5a-d show the mechanical response of enantiomeric molecules. a, Biomechanical forces arising from D-VSR (--3800 pN) and L-VSR (~-200 pN) coated cantilevers against 50 $\mu$M Van in sodium phosphate buffer pH 7.4 b, Corresponding semi-logarithmic plot of biomechanical forces as a function of Van concentration. c, Mechanical forces generated from D-VSR (--5000 pN) and L-VSR (--2000 pN) coated cantilevers against 50 $\mu$M Rist in sodium phosphate buffer pH 7.4. d, Corresponding semi-logarithmic plot of biomechanical forces as a function of Rist concentration. In a and c, the shaded areas represent the time frame during which sodium phosphate buffer without ligands was injected for 10 min in order to establish a baseline and the downward bending of the cantilever corresponds to compressive biomechanical force on the Au surface. The black line represents the mechanical force response from the control PEG coated cantilevers. In b and d, the error bars shown represent the standard deviation of the mechanical force response. The solid lines connecting the solid and open squares represent data points for visual guidance only and not fitted to any particular equation. The results demonstrate that biomechanical force is strongly influenced by the degree of complementarity of ligand-receptor complex.

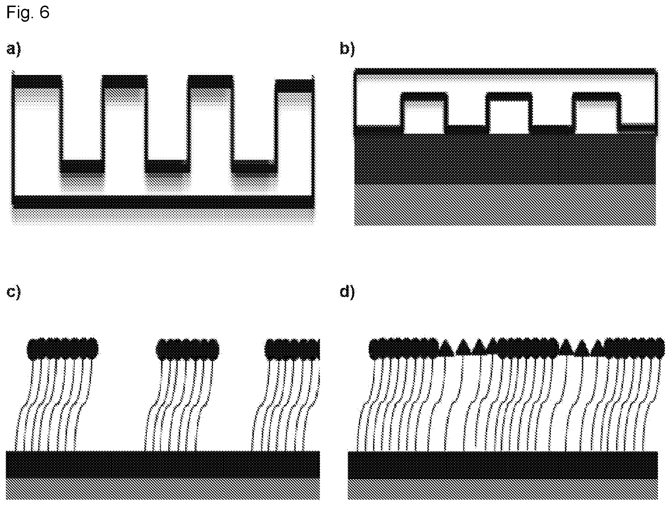

Figures 6a-d show printing of transduction arrays using μCP stamps; a, Schematic representation of a PDMS stamp in which it is inked with self-assembled monolayers (SAMs) of mercaptoundecanoic acid (MUA) (diamond-headed groups). b, The inked PDMS stamp is brought into conformal contact with the Au-coated silicon substrate to enable the SAMs to diffuse from the PDMS stamp onto the Au-surface. c, Schematic representation of MUA arrays arranged on Au-coated silicon substrate generated by micro-contact printing prior to exposure to UDT SAMs. d, The same MUA array following exposure to UDT SAMs in which the un-patterned areas on the Au-coated silicon substrate are passivated with UDT (circle-headed group).

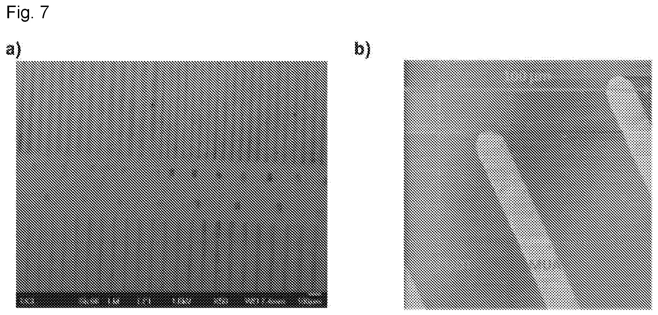

Figures 7a-b show Au-coated silicon substrate printed using a μCP stamp; a, Scanning electron microscopy (SEM) image showing patterns of self-assembled monolayers (SAMs) of mercaptoundecanoic acid (MUA) on Au-coated silicon substrate prepared by micro-contact printing (μCP). Scale bar, 100 μm. b, Atomic Force Microscopy (AFM) image showing SAMs of MUA (square pattern) and undecanethiol (UDT) (un-patterned area) on Au-coated silicon substrate prepared by μCP. Scale bar, 10 μm.

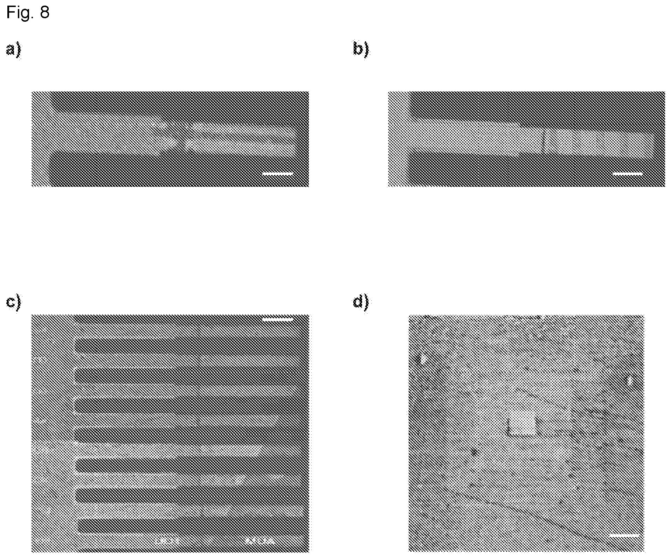

Figures 8a-d show the impact mechanical connectivity has on signal response; a, SAMs of MUA array networks arranged as a narrow strip (solid line) running centrally along the entire length of the cantilever and continuous with the hinge region. b, MUA SAMs arranged in strips transverse to the long axis of the cantilever (solid lines) creating mechanical networks which are discontinuous with each other and with the hinge region. c, Array networks of MUA SAMs arranged continuously from the free end of the cantilever but terminating at various distances from the hinge region. In a-c, the un-patterned areas on the cantilever (light background) were passivated using UDT SAMs to block nonspecific interactions. d, Atomic Force Microscopy (AFM) image of MHA SAM patterns (central light square) on a Au-coated silicon substrate prepared by dip-pen nanolithography (DPN). Scale bar, 1 μm.

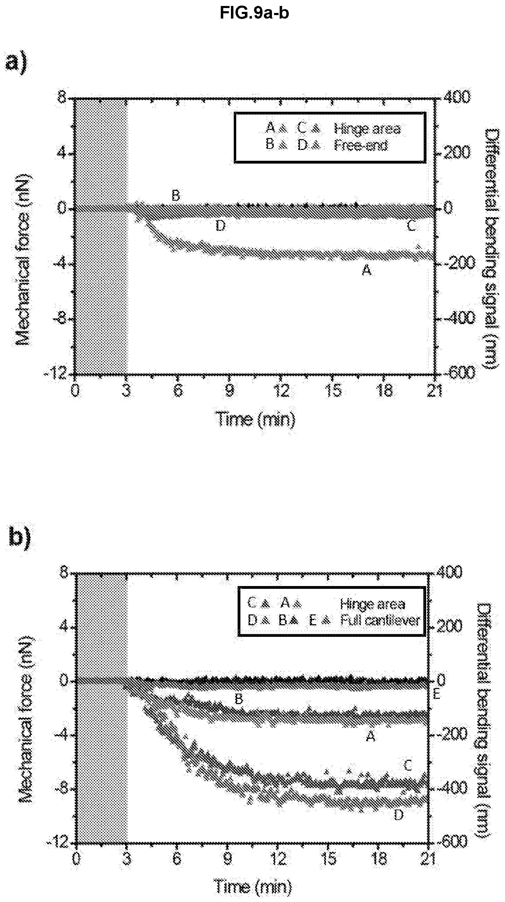

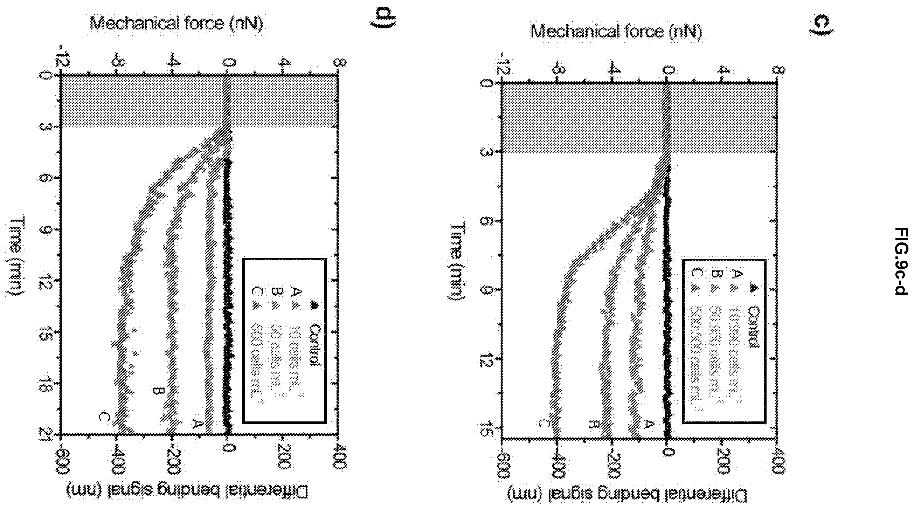

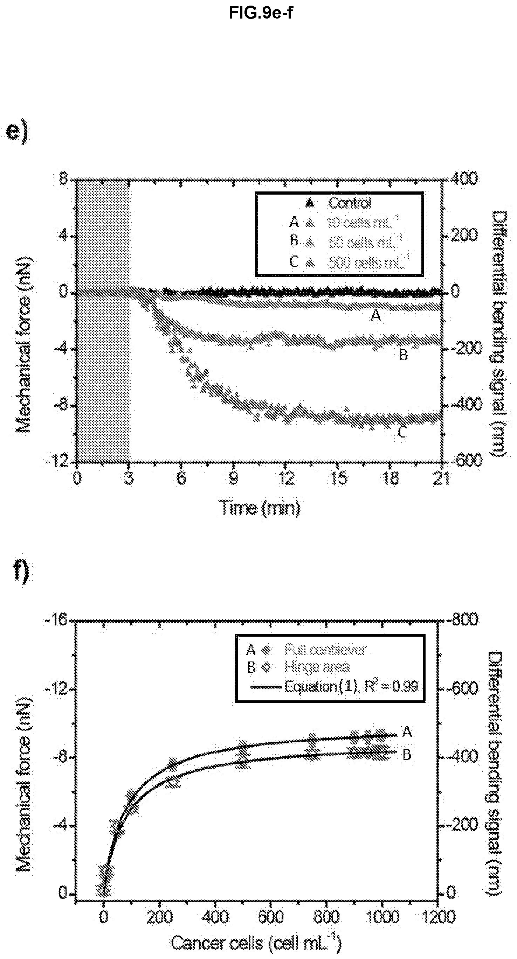

Figures 9a-f show the mechanical connectivity in mechanobiology and cell response; a, The mechanical force obtained from 25 cell ml-1 malignant breast cells against receptors at hinge region (large signal response, A) and free-end (small signal response, B). Corresponding mechanical force obtained from 25 cell ml-1 normal mammary epithelial cells against receptors at hinge region (small signal response, C) and free-end (small signal response, D). b, The mechanical force obtained from 25 cell ml-1 malignant breast cells against receptors at hinge region (A) and full cantilever (B). Corresponding mechanical force obtained from 500 cell ml-1 malignant breast cells against receptors at hinge region (C) and full cantilever (D). Corresponding mechanical force obtained from 25 cell ml-1 normal mammary epithelial cells against receptors at full cantilever (E). c, The mechanical force against receptors at hinge region obtained from 10 cell ml-1 malignant breast cells in 990 cell ml-1 normal mammary epithelial cells (A), 50 cell ml-1 malignant breast cells in 950 cell ml-1 normal mammary epithelial cells (B) and 500 cell ml-1 malignant breast cells in 550 cell ml-1 normal mammary epithelial cells (C). d, The mechanical force against receptors at hinge region obtained from 10 cell ml-1 (A), 50 cell ml-1 (B) and 500 cell ml-1 (C) malignant breast cells. e, The mechanical force against receptors at full cantilever obtained from 10 cell ml-1 (A), 50 cell ml-1 (B) and 50 cell ml-1 (C) malignant breast cells using entire cantilever coated with cancer cells targeting peptides. In a-e, the shaded areas represent the 3 min time frame during which sodium phosphate buffer was injected without cells to establish a baseline and the negative signals are associated with compressive force on the sensing element. The reference signal is shown and named "Control". f, A plot showing the measured mechanical force obtained from malignant breast cells using entire cantilever coated with cancer cells targeting peptides (solid diamond) and at hinge region (open diamond). The data are described by Eq. (1) for Kd = 81 ± 4 cell ml-1 (solid diamond, full cantilever, A) and for Kd = 80 ± 5 cell ml-1 (open diamond, hinge region, B). The error bars shown represent the standard deviation of the mechanical force obtained from four separate chips. This shows for the first time that connectivity of receptors with each other and with the hinge region can be applied in mechanobiology and cell response to quantify mechanical signaling.

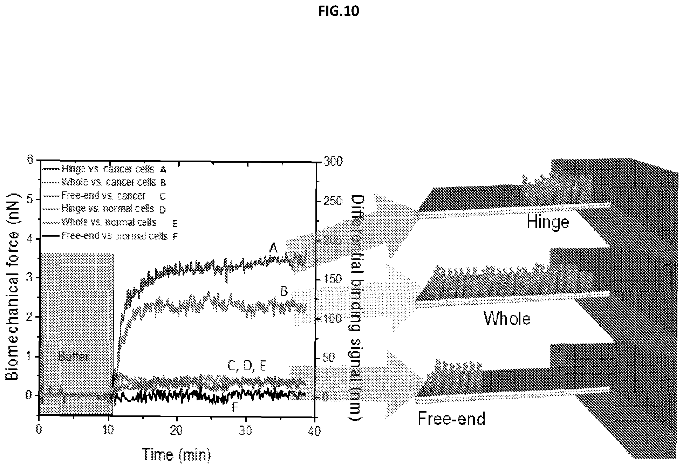

Figure 10 show the impact of receptor location on mechanical signaling and the differential binding force of the receptor (peptide 18-4) to cancer cells while it is coated on the cantilever in three different geometrical fashions with respect to the control experiment performed with normal epithelial cells (MCF10A). In particular: the mechanical force obtained from 25 cell ml-1 malignant breast cells against receptors at hinge region (large signal response, A), the mechanical force obtained from 25 cell ml-1 malignant breast cells against receptors with full cantilever (B), the mechanical force obtained from 25 cell ml-1 malignant breast cells against receptors at free-end (small signal response, C), the corresponding mechanical force obtained from 25 cell ml-1 normal mammary epithelial cells against receptors at hinge region (small signal response, D), full cantilever (small signal response, E) and free-end (small signal response, D). The shaded areas represent the 3 min time frame during which sodium phosphate buffer (pH 7.4) was injected to establish a baseline.

## Detailed description of the disclosure

"Cantilever array" or "array of cantilevers" means a display comprising a plurality of cantilevers, preferably at least 8 cantilevers, anchored at only one end to a support from which they protrude.

"Hinge region" means the anchoring area between one end of the cantilever and a support.

"Continuous connectivity" means that the distance (i.e. the length) between the plurality of probe molecules composing the SAM is equal or less than 50 μm and that the distance (i.e. the length) between the SAM network and the hinge region is equal or less than 50 μm.

"Self-assembled monolayer (SAM) of a probe molecule" or "network of probe molecules" or "SAM network" means a plurality of probe molecules each one connected to the surface of a cantilever through a linker.

The present disclosure refers to a cantilever sensor comprising a silicon layer coated on one side (or surface) with a coating comprising Au and an end region anchored to a support, thereby forming a hinge region. The Au-coated surface is further coated with a self-assembled monolayer network of probe molecules that covers between 30% and 80% of the Au layer surface and is arranged along the longitudinal length of the cantilever or transverse to the long axis of the cantilever in a continuous connectivity between the probe molecules of the network and between the network and the hinge region of the cantilever.

The continuous connectivity is obtained when the distance between a self-assembled monolayer network of probe molecules and a hinge region of the cantilever is equal or less than 50 μm and when the distance between the plurality of probe molecules is equal or less than 50 μm.

According to the present disclosure (not part of the present invention), the self-assembled monolayer network of probe molecules covers between 30% and 100% of the Au layer surface. According to the present disclosure (not part of the present invention), the self-assembled monolayer network of probe molecules covers less than 100% of the Au layer surface.

According to the present disclosure (not part of the present invention), the self-assembled monolayer network of probe molecules covers between 30 and 90%.

In one embodiment of the present invention, the self-assembled monolayer network of probe molecules covers between 30 and 70%, or between 30 and 60% or between 30 and 50%, or between 30 and 40% of the Au layer surface. The continuous connectivity, the coverage percentage and the disposition along the longitudinal length of the cantilever of the probe molecule network and continuity with the hinge region are important parameters in improving the reproducibility of the signal response.

The cantilever can be unpassivated on the opposite side (or surface) of the Au layer or, alternatively, the cantilever can be passivated with, for example, a PEG-silane coating.

The probe molecule is able to bind in a selective way to the molecule to be detected in a body fluid, thereby forming a bound complex that causes the physical deflection of the cantilever. Bending of the cantilever caused by the biomechanical forces generated from molecular interactions can be detected and correlated to the molecule concentration in the body fluid.

A cantilever surface can be coated with a first layer of titanium (called adhesion layer) on top of which the Au layer is deposited.

The thickness of the titanium layer is between 1 and 5 nm. The thickness of the gold layer is between 5 and 50 nm, preferably between 10 and 20 nm.

The cantilever sensor of the present disclosure is preferably included in a sensor array comprising at least 8 cantilevers or more. Each cantilever is anchored at only one end to a support from which it protrudes. The anchoring area is called hinge region.

The cantilever of the disclosure is able to detect the presence of a molecule in a body fluid with improved limits of detection in sub-femtomolar quantities without compromising signal reproducibility or the need to use sample labeling.

Preferably, the cantilever of the disclosure has a rectangular shape. Typical sizes of the cantilever sensor are: 500 μm long, 100 μm wide and 1 μm thick. According to a preferred embodiment of the invention, the width of the cantilever can be tuned based on the needs of the limits of detection sensitivity as well as the standard instruments of the detection system and the interplay with the capillary forces as well as the static charges which may render handling more difficult. In addition, the present disclosure refers to the use of the width and length of the regions covered by probe molecules to control the size of the sensing element and, ultimately, to enhance the limits of detection for ligands. The preferred width of the cantilever is between 30 and 100 μm, preferably between 70 and 100 μm. Such a narrow configuration of the cantilever geometry could significantly improve the limits of detection by $10^4$-fold without compromising signal reproducibility or the need to use sample labeling.

The coating of one side of the cantilever with at least a gold layer preferably includes the deposition of a first (or base) titanium layer (called the adhesion layer) and then a top gold layer.

The Au coating is achieved by using any method known in the art. Preferably, the Au coating is prepared by using any of the known physical thermal vapor deposition (PVD) methods (for example, the thermal evaporation technique) or any of the known PVD techniques, such as the electron beam evaporation technique.

Metal coating preparation includes deposition of a first titanium layer followed by deposition of a gold layer under vacuum until the desired thickness is achieved. Typically, the thickness of the titanium layer is between 1 and 5 nm. The thickness of the gold layer is typically between 5 and 30 nm, preferably between 10 and 20 nm.

Self-assembled monolayer (SAM) refers to organic molecule assemblies that form spontaneously on surfaces (for example by adsorption) and are organized into more or less large ordered domains. Typically, molecules that assemble into monolayer possess a head group that has a strong affinity to the surface and anchors the molecule to it, a tail and an end functional group. Common head groups include thiols, silanes, phosphonates, etc.

The self-assembled monolayer of the disclosure preferably is an alkanethiol self-assembled monolayer in which the alkanethiol moiety is the linker between the probe molecule (i.e. functional group) and the Au and/or Si surface, as depicted below:

| Au and/or Si surface | -linker- | probe molecule |
|---|---|---|

Preferably, the alkanethiol linker is an alkanethiol polyethylene glycol moiety.

The linker interacts with the Au and/or Si surface through the terminal -SH residue and is covalently attached to the probe molecule through the -OH group of the polyethylene glycol moiety. The interaction between the Au and/or Si surface and the alkanethiol linker is a semi-covalent type of interaction due to the strong affinity of sulfur for these metals.

In a preferred embodiment, the alkanethiol linker is $HS(C_{8-15})alkyl-(OCH_2CH_2)_nOH$, wherein n = 2-5.

Preferably, the alkanethiol linker is $HS(OCH_2CH_2)_nOH$ wherein n = 3 or 6, that binds to the Au and/or Si surface of the cantilever via the -SH residue and to the probe molecule via the -OH group, as depicted below:

| Au and/or Si surface | $-S(OCH_2CH_2)_nO-$ | probe molecule |
|---|---|---|

The probe molecule can be any molecule able to interact with specificity and sensitivity with another molecule (a coupling molecule), thus generating ligand-receptor or drug-receptor or sequence-specific DNA or RNA hybridization-type interactions or antibody-antigen interactions.

The probe molecule preferably is a receptor able to provide ligand-receptor or drug-probe binding or a probe molecule able to selectively hybridize to a complementary DNA or RNA sequence or an antibody able to provide antibody-antigen interaction.

For example, the receptor is selected from: the vancomycin susceptible receptor (VSR), monoclonal human immunodeficiency virus antibody (anti-p24), factor (VIII) antibody (anti-Factor (VIII)), polyclonal anti-prostate-specific antibody (anti-PSA), anti-pancreatic stone protein (anti-PSP), soluble CD4 antibody (anti-CD4), anti-immunoglobulin G (anti-IgG) antibody, cancer cell targeting peptide or combinations thereof.

The coupling molecule is a ligand, a drug molecule, a protein, an antigen, a hybridizing nucleic acid sequence, a human cell or combinations thereof. For example, the coupling molecule is vancomycin (Van), pancreatic stone protein (PSP), CD4T cells, glycoprotein p24, factor (VIII), immunoglobulin G (IgG), intact cells, microRNAs, prostate specific antigen (PSA), circulating cancer cells, such as breast cancer cells or mammary epithelial cells.
The probe molecule is attached with the linker, preferably an alkanethiol linker, prior to the preparation of the incubating solution.

To coat the cantilevers with probe molecules at a distance that is less than 50 $\mu$m among the SAMs and the hinge region the dip-pen lithography (DPN) and micro-contact printing ($\mu$CP) methods were used. This is because these methods have the ability to write patterns prescribing two-dimensional (2D) assembling of SAMs with sub-micron precision. The cantilevers were printed on the Au-coated surface using SAMs of probe molecules as the printing ink. To print SAMs on the cantilevers, for example a poly(dimethylsiloxane) (PDMS) stamp was first impregnated with SAMs of probe molecules by incubating for 1-5 minutes, preferably 1-2 minutes. Excess SAMs solution was removed from the stamp by blowing nitrogen gas or similar gas over the stamp. The impregnated stamp was then placed in a conformal contact with Au-coated surface of the cantilever for 2 min where gentle pressure (using a one penny coin) was applied on the stamp to allow close contact with Au surface so that the SAMs could diffuse from the stamp onto the substrate and enable uniform molecular printing. The printing of cantilevers was carried out in an upside down configuration in which the stamp faced upwards whereas the Au-coated surface faced downwards. The stamp was removed after 1-5 minutes and the un-patterned areas on the Au-coated surfaces were then blocked with non-specific SAMs such as undecanethiol (UDT),hexadecanethiol (HDT) or 6-mercapto 1-hexanol (MCH), which do not couple with molecules of interest (Figures 6,7). In case of DPN, the printing process involved using a sharp scanning cantilever tip to transfer SAMs of probe molecules as the printing ink directly onto the designated surface. The cantilever tip functionalized with SAMs of probe molecules was brought into contact with the Au-coated cantilever surface and the desired patterns of receptor was slowly traced for example at a resolution of about 256 lines per $\mu m^2$ and at a frequency of about 1 Hz. The low scan speed was necessary to enable precise delivery of probe molecules to the surface via formation of a liquid meniscus. Following this, the un-written areas on the cantilevers were then blocked with non-specific SAMs (Figures 6-8). Working with the μCP and DPN described above ensured that only SAM are formed on the Si top Au-surface while at the same time limiting any deposition on the Si bottom side of the cantilever. Accordingly, the influence of the negative contributions of the interaction between the SAM formed on the Si side of the cantilever and the coupling molecule to the overall stress signal is completely eliminated.

In the case the cantilever is passivated on the side opposite to the gold layer (i.e. bottom side), a PEG-silane coating is applied on the bottom side of the cantilever. The purpose of passivation of the bottom surface is to help in avoiding unwanted functionalization of the bottom surface with receptors or probe molecules, consequently preventing probe molecule (ligand) adsorption that would alter sensing results.

The present disclosure refers also to a method for detecting the presence of a ligand/drug molecule in an *ex-vivo* body fluid, such as blood, plasma, urine, saliva, sweat or sputum (or combinations thereof), comprising the steps of:

1) Providing a cantilever sensor according to the present disclosure;
2) Contacting the cantilever with a body fluid containing the molecule to be detected;
3) Detecting the response signal due to cantilever bending;
4) Correlating the response signal to the presence or absence of the ligand to be detected and, in case of presence, to the concentration of the ligand in solution.

The cantilever sensor can be included in an array of at least 8 unpassivated cantilevers or more.

The contact between the cantilever and the body fluid in step 2) is performed for a period of about 5 - 30 min, although shorter or longer times can be used.

During the contact, the probe molecule selectively binds to the ligand to be detected via a receptor-ligand, receptor-drug, antibody-antigen or hybridizing sequence-type of interaction, thereby forming a complex that creates biomechanical force and consequently a cantilever bending response that can be detected.

Detection of cantilever bending response in case of optical readout is performed, for example, by using serial time multiplexed optical beam method with a single position sensitive detector, although other readouts such as electronic or diffraction can be used. The laser spot (about 100 μm diameter) is aligned onto the free end of each sensor where the accuracy of alignment is confirmed by heating test. The expected precision of laser spot alignment is determined by calculating the bending variation at the maximum bending signals between individual cantilevers. Well aligned cantilevers at the maximum bending signals should yield a relative standard deviation of the bending signals of about ≤10%, preferably about ≤5%, between them. Correlating the response signal to the presence or absence of the ligand to be detected includes a first step of calculating the net change in biomechanical force, using the mathematical model reported in equation (2), and then a second step of associating the net change in biomechanical force value to the presence or absence of the molecule and, in case of presence, to the concentration level of the ligand.

The molecule is considered not present in the body fluid when the measured differential biomechanical force is equal to about zero or very small, which corresponds to the physical cantilever bending of about zero or very small. As understood by one skilled in the art, the absence of a substance from a solution means that the substance concentration is below the limits of detection of the analysis method. For the present method, a ligand to be detected is considered absent from a body fluid when the concentration of such a ligand is below the current limits of detection in femtomolar or even sub-femtomolar quantity.

A ligand is considered present in a body fluid when the induced biomechanical force net signal is ≥ 20 pN.

**EXPERIMENTAL PART**

**Methods and materials**

**Two-dimensional assembling of ligand-presenting molecules**

To test the hypothesis that mechanical forces arising from ligand-receptor binding interactions can be amplified to enable measurements of biologically relevant signals with high precision, different patterns were designed with a prescribed 2D-assembling of transduction arrays. The dip-pen lithography (DPN) and micro-contact printing (μCP) were used to create arrays parallel or transverse to the long axis of the conventional Au-coated nanomechanical biosensor

arrays of (100 μm wide and 500 μm long IBM Rushlikon). The DPN has the advantage of enabling receptor patterns to be fabricated with nanometre precision[30-32] whilst μCP allows fabrication to be achieved more rapidly in a single step[33]. Parallel arrays were arranged as a 30 μm or 50 μm wide strip running centrally along the entire length of nanomechanical biosensor arrays covering 30% or 50% of the total surface area, respectively. Transverse arrays were arranged as 30 μm wide strips running along the width of the cantilever arrays, and corresponding to 30% of the total surface area. A control cantilever array had 100 μm wide strips running centrally along the entire of their, and corresponding to 100% of the total surface area. SAMs of mercaptoundecanoic acid (MUA) and mercaptohexadecanoic acid (MHA) were used to create transduction arrays. MUA was used to create 30 μm wide strips for both parallel and transverse arrays as well as for control cantilevers. MHA was used to create 50 μm strips in the parallel arrays as well as for control cantilevers (see, fabrication of transduction arrays by μCP stamps and DPN).

**Cantilever and silicon substrate preparation**

Silicon substrates measuring 1 cm × 1 cm each were cleaned by incubating them in freshly prepared piranha solution, consisting of $H_2SO_4$ and $H_2O_2$ (1:1) for 20 min. They were then briefly rinsed in ultrapure water followed by rinsing in pure ethanol before being dried on a hotplate at 75 °C. The substrates were then examined under an optical microscope to confirm their cleanliness before being transferred to an electron beam evaporation chamber (BOC Edwards Auto 500, U.K.) where they were coated at a rate of 0.7 $nms^{-1}$ with a 2 nm layer of titanium, which act as an adhesion layer, followed by a 20 nm layer of Au. Once the required thickness of Au was obtained, the silicon substrates were left in the chamber for 1-2 h to cool under vacuum. Au-coated atomic force microscopy (AFM) cantilevers were prepared using the same procedure.

**Fabrication of micro-contact printing stamps**

Silicon master moulds were prepared using standard photolithography. Micro-contact printing (μCP) stamps made of poly(dimethylsiloxane) (PDMS) were prepared from the moulds. Poly(dimethylsiloxane) was chosen because after polymerization and cross-linking, the solid PDMS is highly flexible and easy to peel off Si or Au-coated surfaces. PDMS polymer solution was freshly prepared by mixing a silicone elastomer 184 base and silicone elastomer 184 curing agent (Dow Corning Corporation, USA) at a ratio 10:1 by weight in a disposable plastic container. The plastic container was then placed in a glass beaker inside a vacuum dessicator for 20 min to remove any gas bubbles. The resulting viscous solution was poured over the silicon master mould and baked in a 75 °C oven for 1 h to enable cross-linking of the polymer and transfer of the etched pattern from the silicon master onto solid PDMS. After cooling the imprinted solid PDMS stamps were peeled away from the silicon master and trimmed to the required size.

**Printing of transduction arrays using μCP stamps**

Transduction arrays were printed on to Au-coated silicon substrates and cantilevers using self-assembled monolayers (SAMs) of MUA as the printing ink. MUA was chosen as it is an alkanethiol with only 11 carbons and thiolates with less than 20 carbons are known to enable the production of stable printing patterns with defined boundaries. In addition MUA SAMs can be attached to variety of receptor molecules allowing the detection of a variety of receptor targets. In this study MUA was attached to the vancomycin-susceptible receptor (VSR) and anti-immunoglobulin G (anti-IgG) antibody to enable detection of vancomycin (Van) and antigen immunoglobulin G (IgG) respectively.

The protocol for printing MUA SAMs onto Au-coated silicon substrates and cantilevers is summarized in Fig. 6. The PDMS stamp was first cleaned by rinsing in pure ethanol before it was dried under nitrogen gas. The stamp was then impregnated with MUA by incubating it in a freshly prepared solution of MUA in ethanol at a total SAM concentration of 2 mM for 1 min. Excess MUA solution was removed from the PDMS stamp by blowing nitrogen gas over the PDMS stamp. The impregnated stamp was then placed in conformal contact with Au-coated surface for 2 min where gentle pressure (using a one penny coin) was applied on the PDMS stamp to allow close contact with Au surface so that the MUA SAMs could diffuse from the PDMS stamp onto the substrate and enable uniform molecular printing. The printing of cantilever chips was carried out in an upside down configuration in which the PDMS stamp faced upwards whereas the Au-coated cantilever surface faced downwards. The cantilever, which was initially oriented with an angle of tilt away from the surface horizontal, was carefully moved downwards until it was in full contact with the surface of the PDMS stamp. The PDMS stamp was removed after 2 minutes and the un-patterned areas on the Au-coated surfaces were then blocked with non-specific SAMs such as undecanethiol (UDT) or hexadecanethiol (HDT) which do not couple with molecules of interest. This was followed by a rinse in pure ethanol and then dried under nitrogen gas. Plain, un-patterned PDMS stamps were used to print the continuous mechanical networks which terminated at varying distances from the hinge region or free end of the cantilever. Because of the fragile nature of cantilevers, the PDMS stamps were used to print SAMs of MUA onto the Au-coated AFM cantilevers only after mastering the procedure and if the printing was deemed satisfactory with the Au-coated silicon substrates.

The accuracy of the printed patterns on the Au-coated surfaces was checked by scanning electron microscopy (SEM). Fig. 7a shows a typical image of the MUA pattern printed on a Au-coated silicon substrate. The printed pattern was further examined using Atomic force microscopy (AFM) as shown in Fig. 7b. In addition, the Au-coated surfaces were exposed to moist air which preferentially condenses on to the MUA coated surfaces. When viewed under a light microscope the hydrophilic MUA coated areas appeared dark in contrast to the hydrophobic UDT coated areas (Fig. 8a-c).

**Dip-pen nanolithography**

SAMs of mercaptohexadecanoic acid (MHA) were printed onto Au-coated silicon substrates and cantilevers using dip-pen nanolithography (DPN). This printing process involved using a sharp scanning AFM cantilever tip to transfer the MHA as the printing ink directly onto the designated surface and create the desired patterns. The success of this approach was first tested on the Au-coated silicon substrates (Fig. 8d). An AFM cantilever tip functionalized with MHA at a total SAM concentration of 2 mM was brought into contact with the Au-coated silicon substrate and a 1$\mu$m square pattern was slowly traced at a resolution of 256 lines and at a frequency of 1 Hz. The low scan speed was necessary to enable precise delivery of MHA to the surface via formation of a liquid meniscus. The printed pattern was then imaged using the same AFM cantilever tip where a large area of 10 $\mu$m square was scanned at the same resolution but using a scan speed of 10 Hz. The high scan speed was essential to prevent deposition of any additional MHA during imaging. Fig. 8d shows the MHA pattern on a Au-coated silicon substrate which is clearly distinct from the underlying un-patterned areas.

Having established that DPN could be successfully used to create array networks on Au-coated silicon substrates, the same procedure to create transduction arrays on the cantilevers was then applied. Following this, the unpatterned areas on the cantilever were then blocked with non-specific SAMs such as undecanethiol (UDT) or hexadecanethiol (HDT) which do not couple with molecules of interest. Although DPN enables the creation of transduction arrays with high resolution, this process is relatively laborious and time consuming. The future challenge therefore is to find ways of making the process quicker and more efficient.

**Synthesis of vancomycin-susceptible receptors (D-VSR and L-VSR)**

**D-VSR** and **L-VSR** peptide synthesis was carried out as previously described[11,3]. The cleaved products were purified using reverse phase HPLC by varying the mobile phase from 5% to 95% acetonitrile in water (with 0.5% trifluoroacetic acid). The peptides were characterized by NMR and HRMS (+ESI, Q-TOF) as follows;

i) D-Ala-OH (HS(CH2)$_{11}$(EG)$_3$-OCH$_2$-Ahx-L-Lys-D-Ala-D-Ala-OH

$^1$H NMR ($\delta$ ppm, 400 mHz, $CD_3OD$): 1.25-1.80 (m, 36H, [15CH$_2$ + 2 CH$_3$]), 1.91 (s, 3H, CH$_3$), 2.24 (t, 2H, CH$_2$), 2.47 (t, 2H, J = 7.1 Hz, C*H*$_2$SH), 3.14 (t, 2H, J = 7.0 Hz, NHC*H*$_2$), 3.22 (t, J = 7.0 Hz, 2H, NHC*H*$_2$), 3.44 (t, 2H, J = 6.7 Hz, -C*H*$_2$(OEG)-), 3.54-3.69 (m, 12H, 3(EG)), 3.96 (s, 2H, OC*H*$_2$C=O), 4.21 (t, 1H, J = 7.1 Hz, L-lys-$\alpha$-C*H*), 4.31-4.42 (m, 2H, 2 [D-Ala-$\alpha$-C*H*]).

$^{13}$C NMR ($\delta$ ppm, 125 mHz, $CDCl_3$): 176.24, 175.58, 174.46, 174.29, 173.21, 172.61 (6 C=O), 72.39, 71.95, 71.55, 71.52, 71.37, 71.22, 71.16 (PEG), 55.15, 50.00, 40.17, 39.83, 36.50, 35.22, 32.27, 30.72, 30.70, 30.64, 30.56, 30.27, 30.21, 29.40, 27.57, 27.21, 26.46, 24.97, 24.23, 22.57, 18.01, 17.50.

HRMS (+ESI, Q-TOF), found 842.4941; required for $C_{39}H_{73}N_5O_{11}SNa$ [M+Na]+, 842.4925, dev. 1.86 ppm.

ii) L-Ala-OH (HS(CH2)$_{11}$(EG)$_3$-OCH$_2$-Ahx-L-Lys-D-Ala-L-Ala-OH

$^1$H NMR ($\delta$ ppm, 400 mHz, $CD_3OD$): 1.25-1.80 (m, 36H, [15CH$_2$ + 2 CH$_3$]), 1.91 (s, 3H, CH$_3$), 2.24 (t, 2H, J = 7.2 Hz, C*H*$_2$), 2.47 (t, 2H, J = 7.1 Hz, C*H*$_2$SH), 3.15 (t, 2H, J = 7.0 Hz, NHC*H*$_2$), 3.22 (t, J = 7.0 Hz, 2H, NHC*H*$_2$), 3.45 (t, 2H, J = 7.0 Hz, -C*H*$_2$(OEG)-), 3.54-3.67 (m, 12H, 3(EG)), 3.96 (s, 2H, OC*H*$_2$C=O), 4.14 (t, 1H, J = 7.1 Hz, Lys-$\alpha$-C*H*), 4.31-4.40 (m, 2H, 2 [Ala-$\alpha$-C*H*]).

$^{13}$C NMR ($\delta$ ppm, 125 mHz, $CDCl_3$): 176.22, 175.83, 174.67, 174.44, 173.21, 172.62 (6 C=O), 72.39, 71.96, 71.57, 71.53, 71.39, 71.22, 71.18 (PEG), 55.52, 50.24, 40.16, 39.82, 36.39, 35.24, 32.00, 30.74, 30.72, 30.65, 30.58, 30.28, 30.22, 30.06, 29.41, 27.58, 27.22, 26.45, 24.97, 24.28, 22.57, 17.93, 17.60.

HRMS (+ESI, Q-TOF), found 842.4916; required for $C_{39}H_{73}N_5O_{11}SNa$ [M+Na]+, 842.4925, dev. -1.11 ppm.

**Synthesis of cancer cells targeting peptide (18-4)**

**Peptide (18-4)** is a short decapeptide that was engineered from the cancer homing peptide P160 to be enzymatically stable in biofluidic environments. The synthesis of peptide (18-4) whose details of synthesis has been described previously[43] was thiolated with a cysteine residue. It was isolated and selected by RP-HPLC retention time (Rt) of synthetic peptides measured using Vydac C18 analytical column with a gradient of (mobile phase 1 for peptides 1 and 2) 15 - 50% ACN/water (0.1% TFA) in 50 min with a flow rate of 1 ml $min^{-1}$ or (mobile phase 2 for peptides 3 and 4) 15 - 55% ACN/water (0.1% TFA) in 50 min with a flow rate of 1 ml $min^{-1}$.

**Cantilever functionalization with surface target molecules**

i) **D-VSR and L-VSR**

In the case of preparation of unpassivated cantilever, SAMs of D-VSR, L-VSR and inert PEG molecules were diluted to a concentration of 1 μM in pure ethanol. The diluted SAMs were individually injected into glass capillary tubes (King Precision Glass, Claremont, CA, USA) which were arranged on the functionalization stage. The cantilevers were then functionalized by incubating them with individual SAMS inside the glass capillaries for 20 min. Care was taken to ensure that the hinge region was entirely covered with the SAM solution in order to ensure mechanical connectivity between the transduction arrays and hinge region.

ii) **Anti-mouse IgG functionalization**

In the case of preparation of passivated cantilever, Au-coated silicon cantilevers were functionalized with anti-mouse immunoglobulin G (anti-IgG) antibody (Sigma-Aldrich, UK) as follows: The cantilevers were inserted into the glass capillary tubes containing a mixture of ethanolic thiol solutions of PEG (HS-C11-$(Eg)_3$-OMe) and NHS (HS-C11-$(Eg)_3$-$OCH_2$-COONHS) (where Eg is an ethylene glycol group, Me is a methyl group and NHS is the N-Hydroxysuccinimide group). PEG and NHS thiol solutions were mixed at a ratio of 1:9 respectively to yield a total concentration of 1 μM in pure ethanol before injecting into the glass capillaries. Cantilevers were placed inside the PEG /NHS containing glass capillaries for 20 min then rinsed in pure ethanol and dried under nitrogen gas for 2 min. To passivate the underlying Si surface, the PEG /NHS coated cantilevers were incubated in 2-[methoxypoly(ethyleneoxy) propyl]trimethoxysilane for 30 min followed by a rinse in pure ethanol. They were then activated by placing them in sodium acetate buffer (5mM, pH 5.4) for 5 min at room temperature. They were removed from the sodium acetate buffer and placed inside a chamber where they were submerged in a droplet containing 100 μg/ml of anti-IgG antibody dissolved in phosphate-buffered saline solution (PBS) pH 7.4. The chamber was then kept at 4°C overnight to enable complete conjugation of NHS and IgG. To cap any unconjugated NHS molecules, the cantilevers were incubated in 1 M ethanolamine pH 8.5 for 5 min at room temperature. This was followed by a wash in PBS buffer pH 7.4. The freshly functionalized cantilevers were either used immediately or stored in PBS at room temperature for later use.

iii) **Cancer targeting peptides and inert 6-mercapto 1-hexanol (MCH)**

SAMs of MCH and cancer targeting peptides (18-4), were diluted in pure ethanol to yield a total concentration of 1 mg $ml^{-1}$. To functionalize the hinge area with the cancer targeting peptides, the free-end of nanomechanical cantilever arrays was first coated with 1 mg $ml^{-1}$ SAMs of MCH solution to prevent nonspecific adsorption of cells on the Au-coated surface. The incubation lasted for 10 min, and the cantilever chip was rinsed with phosphate-buffered saline (PBS) solution at pH 7.4 before drying in air. Following this step, the un-patterned hinge area of the nanomechanical cantilevers was functionalized with cancer targeting peptides by incubating the entire cantilever chip in 1 mg $ml^{-1}$ SAMs of cancer targeting peptide for 20 min followed by a rinse in PBS solution. Similarly, to functionalize the free-end of nanomechanical cantilever arrays with cancer targeting peptides, the hinge area was first coated with SAMs of MCH solution for 10 min, rinsed in PBS solution and dried in air. This was followed by incubating the cantilever arrays in 1 mg $ml^{-1}$ SAMs of cancer targeting peptide for 20 min and rinsed in PBS solution. Finally, to functionalize the entire cantilever with cancer targeting peptides, each of the cantilever arrays was incubated in 1 mg $ml^{-1}$ SAMs of cancer targeting peptides for 20 min followed by a rinse in PBS solution. In parallel, the reference cantilevers were also functionalized by incubating them in 1 mg $ml^{-1}$ SAMs of MCH for 20 minutes and rinsed with PBS solution.

**Reagent preparation**

Phosphate buffered saline powder (Sigma-Aldrich, UK) was dissolved in one litre of ultrapure water (18.2 MΩ cm 10 resistivity, Millipore Co., Billerica, MA, U.S.A.) to give a final solution at pH 7.4. Stock solutions of 1 mg/ml IgG antibody and 1.4 mg/ml vancomycin were prepared using the freshly prepared and filtered PBS solution. Serial dilutions of the stocks in PBS were then used to prepare the working solutions of the ligands.

**Impact of a hinge region on signal reproducibility**

Different patterns and sizes of transduction arrays and their impact on signal response were examined. Dip-pen lithography (DPN) and micro-contact printing (μCP) were used to create patterns of transduction arrays parallel or transverse to the long axis of conventional Au-coated cantilevers (100 μm wide and 500 μm long IBM Rushlikon cantilevers). Parallel arrays were arranged as a 30 μm, 50 μm or 100 μm wide strip running centrally along the entire length of the cantilever covering 30%, 50% or 100% of the total surface area, respectively. Transverse arrays were arranged as 30 μm wide strips running along the width of the cantilever, separated by 100 μm and corresponding to 30% of the total surface area. A control cantilever with 100% array coverage was created using glass capillary immobilization. SAMs of MUA and MHA were used to create the trasduction arrays. These SAMs were initially used due their versatility in terms of the diversity of receptors that they can be attached to and for their ability to change surface charge under different pH conditions. When the pH of the surrounding solution is above the isoelectric point (pI ~6)[35] of SAMs, the carboxylate groups become negatively charged due to the loss of protons ($H^+$). Consequently, the repulsive electrostatic interactions between neighbouring negatively charged carboxylate groups create a compressive biomechanical force on the Au surface, causing the cantilever to bend downwards. MUA was used to create the 30 μm wide strips for both parallel and transverse arrays as well as for control cantilevers. MHA was used to create the 50 μm strips in the parallel arrays as well as for control cantilevers. To block nonspecific interactions, the un-patterned areas on the cantilever were passivated using SAMs of undecanethiol (UDT) in the case of MUA, and hexadecanethiol (HDT) for MHA.

Figure 2a,b, shows the signal response obtained after the parallel transduction arrays with 30% coverage were deprotonated by first exposing the cantilvers to phosphate-buffered saline solution (PBS) at pH 4.8 for 3 minutes followed PBS at pH 9.0 for 10 minutes. The signal generated from these arrays was unexpecedly high given that only 30% of the total surface area was occupied. The measurements were repeated using the transduction arrays arranged transverse to the long axis of the cantilever to see if they would produce the same mechanical response (Fig. 2c). Surprisingly it was found that the deprotonation of MUA SAMs transverse to the long axis of the cantilever had negligible effect on its bending with the antiparallel arrays (Fig. 2d). The control cantilevers with 100% array coverage, demonstrated a bending response which was 20% higher than from 30 μm wide parallel arrays (Fig. 2e,f). This is not surprising given a large amount of biomechanical force is expected to be generated from a high density of surface transduction receptor arrays. The experiments were repeated using the MHA parallel network arrays with 50% coverage (Fig. 2g). The signal response was found to be substantially high and in agreement with the previous findings[36] (Fig. 2h). Accordingly, the concept that continuous mechanical connectivity networks drive the efficacy of mechanical signaling with respect to surface recepors is demonstrated (Figs. 2a,b,e,f,g,h). This shows that an increase in the geometrical width of the region covered by ligand-presenting molecules is characterized by a rise in biomechanical force provided receptor connectivity is continuous with the hinge region, confirming the assertion that force generation is a collective behaviour of the receptor interactions. These findings demonstrate that connectivity of receptors with each other and with the hinge region has a significant impact on the signal generation and propagation.. To understand these findings, it can be considered that each ligand-receptor complex on a surface also interacts with the neighbouring complexes via steric and electrostatic forces. These short-range forces when continuous with one another and with the hinge region lead to the propagation of the longitudinal force to bring about a mechanical response. In contrast, when the interactions between the neighbouring complexes are decoupled (Fig. 2c,d), the global longitudinal force is significantly diminished to the extent that it is insufficient to propagate a mechanical response. The results therefore show that the mechanical connectivity to a hinge region have significant influences on the elastic deformations of mechanical devices preclamped to a solid support and could be used to predict receptor coating patterns to generate reliable and reproducible signals.

The dynamic behaviour of mechanical stress propagation to control signal response was further investigated using different geometeric patterns of receptor patching to discern and resolve the potential interplay between mechanical networks and pathways coordinating biomechanical force response. First it was investigated whether the geometrical lengths of the regions covered by the arrays starting from the hinge region of the cantilever activates similar global longitudinal force to those starting at the free-end with the corresponding geometeric width fixed at 100 μm (Fig. 3a). The results reveal a relatively large biomechanical response when the geometrical length of the region covered by arrays start from the hinge region and small response if the origin is from the free-end (Fig. 3b). The minimal response of the free-end receptor coated cantilevers, provides strong evidence of geometry specific effects on the mechanical signaling and, correspondingly, validates the conclusion that the hinge region is more sensitive to stress. This means that the efficacy of the signal registered by the transducer can be controlled by tuning the physical location of chemically reacted. To advance the understanding of how mechanical networks interact collectively, rather than in isolation to generate a global biomechanical force, array networks starting at either the hinge region or free-end of the cantilever were grafted continuously for varying geometeric lengths of the regions covered by array networks along the cantilever and analysed for their mechanical response. Figure 3d displays the relationship between mechanical signaling and the array networks starting from the hinge region. With receptors starting at the hinge, upon increasing the fraction of surface coverage from 0 to 10% negligible nanomechanical signal was measured, whereas from surface coverage of 10% to 100% there was an increase in nanomechanical response in direct proportion to the square of the geometric length covered by receptors in accordance with the power law relationship[37]. On the other hand, for receptors starting at the free-end, upon increasing the receptor coverage from 0 to 90% a negligible nanomechanical signal was measured, whereas from surface coverage of 90% to 100% the response was seen to increase exponentially (Fig. 3e,f). This shows that surface stress is transduced collectively when a relatively large surface fraction is occupied. This means that there is a critical minimum distance between the arrays of receptor molecules with each other and with the hinge region which is necessary to yield an observable mechanical signal. For the separation distance (r) < 50 μm long, there is an intensification of both short and long-range interactions leading to an enhanced longitudinal force. In contrast, for *r* > 50 μm long the mechanical network loses connectivity with the hinge region which leads to a breakdown of the short and long range interactions. Consequently, the reproducibility of the signal response is negatively influenced or even totally disrupted. These findings reveal a strong link between continuity of arrays with the hinge region as a key determinant of mechanotransduction which leads to the cantilever bending response. To our knowledge this is the first demonstration of a hinge region as a prerequisite for mechanically targetable effector of elastic deformation to yield a reproducible signal. Further, this approach unambiguously identifies the origin of variation in mechanical signaling and provides evidence that mechanical connectivity to hinge region can be reprogrammed to generate a reproducible and amplified signal response.

**Hinge region connectivity and sensor geometry on signal response**

The finding that a signal response is detectable even when the assembling of array networks is confined to only 30 μm of the geometrical width of the region covered while the corresponding geometeric length is fixed at 500 μm of the cantilever, prompted us to examine whether changing the dimensions of the cantilever itself whilst maintaining the mechanical connectivity of surface receptors has any impact on the signal sensitivity and reproducibility. This is because the dimensions of a sensing element can greatly impact on the signal response in several ways (1) the threshold size of the ligand capture cross-section area that is able to generate mechanical signaling decreases when the size of the sensing element is reduced, (2) the biomechanical force impacting on the sensing element is more pronounced for a narrow sensing element, and (3) the narrower the sensing element, the less rigid it is and the more responsive it will be to molecular forces. Further, continuous mechanical connectivity can be effected by the edging effects at the perimeter of the sensor itself and its impact would be more pronounced for narrower sensing elements (Fig. 1b). The effect on signal response by reducing the conventional cantilever width from 100 μm to 70 μm whilst keeping the length and thickess at 500 μm and 1 μm, respectively, was examined. These dimensions were chosen because they enable the narrowed cantilever to be used with standard instruments without the need for redesigning equipment. In addition, at this width the cantilever is less affected by the capillary forces and static charges which may render careful alignment of the scanning laser at the free-end more difficult. For this experiment vancomycin (Van) as the reporter molecule and vancomycin-susceptible receptor (or VSR)[21] as its target were used. Van is the Food and Drug Administration (FDA)-approved drug of last resort for clinical treatment of MRSA and clostridium difficile infections[38,39]. This particular system was chosen because it gives relatively large signals and it has been previously shown that the quantitative Van-VSR binding constants can be measured[21] and so it can be used to redefine the limit of recognition of biologically relevant systems. The entire surface of the cantilever including the hinge region was coated with VSR using glass capillares (See Cantilever functionalization with surface target molecules). Artefacts that produce non-specific signals were overcome by performing differential measurements where the reference mercaptoundecyl tri(ethylene glycol) thiol (PEG)-coated cantilever signals was subtracted from the VSR signals. Figure 4a shows the signal response obtained from three separate 70 μm wide cantilevers when they were exposed to 500 pM ($7 \times 10^{-10}$ g ml$^{-1}$) of Van in PBS solution at physiologically relevant pH 7.4 under constant lamina flow rates. The signal response obtained from all three cantilevers was highly consistent and almost identical over a 60 min time period. Moreover, the compressive signal response was relatively high averaging -400 ± 30 pN with a signal-to-noise ratio (SNR) of 7. In contrast, no signal response was obtained when the same expriment was repeated on the 100 μm wide cantilvers using 500 pM Van. Remarkably, the detection limits down to $7 \times 10^{-10}$ g ml$^{-1}$ for Van was found to be 4,000 times better resolution than that obtained with the conventional methods such as Roche/Hitachi Cobas systems - currently in hospital use for the detection of antibiotics, which has reported $1.5 \times 10^{-6}$ g ml$^{-1}$ Van. Next, the impact of different concentrations on signal response initially fixed at 20, 50, 500 and 1000 nM (Fig. 4b-d) was examined. The signal response was found to increase with increasing concentration of Van. The thermodynamic pseudo-equilibrium signal response was attained at 60 and 50 min for 20 nM and 50 nM Van, respectively. In contrast, the thermodynamic pseudo-equilibrium was achieved within 6 min or less for Van concentrations ≥ 500nM. Moreover, the signal response was found to be reversible at all concentrations of Van. Figure 4c shows reproducible signal response for Van at 1 μM concentration plotted as a function of time. To quantify the surface binding affinity assays were repeated with a broader range of Van concentrations, 0.0005 μM to 250 μM, using at least four separate chips for each concentration. The signal response reached a plateau at 10 μM Van (Fig. 4e). When the experiments were repeated using the conventional 100 μm wide cantilevers as a control measurement, the signal response for Van at 1 μM was -120 nm (Fig. 4d), which is in agreement with the previous findings[4,21]. However, this is approximately 50% lower than the signal response obtained with the narrowed cantilevers. Remarkably, the signal response with conventional cantilevers was always lower than the value obtained with narrow cantilevers for the same concentration of Van over a wide dynamice range (Fig. 4e).

To test whether the unexpectedly high and reproducible signal with Van could be replicated with other biologically relevant molecules, additional measurements were performed using a mouse antibody known as immunoglobulin G (IgG) which is a major serum antibody responsible for the recognition, neutralization or elimination of foreign invaders including bacteria, fungi and viruses. As with Van, IgG experiments demonstrated high signal reproducibility and sensitivity. A tensile biomechanical force of -200 pN and SNR of 5 was detectable at the ultra low concentration of 0.2 fg $ml^{-1}$ ($2 \times 10^{-16}$ g $ml^{-1}$) IgG. This is significantly higher (over 50,000 times) resolution than that obtained with currenlty used clinical assays for IgG such as the enzyme-linked immunosorbent assay (ELISA), which has reported detection limits of 10 pg $ml^{-1}$ ($1 \times 10^{-11}$ g $ml^{-1}$ or 0.1 pM)[40]. Using different concentrations of IgG (0.2, 0.5 and 100 fg $ml^{-1}$) in PBS solution pH 7.4 over the same time period, the signal response was found to scale with increasing concentration of IgG which is consistent with the Langmuir adsorption isotherm (Fig. 4f). No signal response was obtained using the 100 $\mu$m wide cantilever when the same three IgG concentrations were employed. This indicates that the continuity of receptor connectivity with a hinge region and sensor geometry are needed for enhanced detection limits as well as signal reproducibility.

The observations demonstrating that signal reproducibility is dependent upon continuity with a hinge region but independent of cantilever geometry have so far been purely phenomenological. To confirm these findings mathematically, a model in which the biomechanical force is assumed to scale as the function of ligand concentration was considered. The changes in biomechanical force when ligands in solution react with surface receptors are quantified by the expression

$$\Delta F_{eq} = F_{\max}\left(\frac{[ligand]^n}{K_d^n + [ligand]^n}\right) \quad (1)$$

Here $F_{eq}$ corresponds to the equilibrium biomechanical force, $F_{max}$ is the maximum biomechanical force when all accessible binding sites are fully occupied, $n$ is the stoichiometric coefficient of the reaction and $K_d$ is the surface thermodynamic equilibrium dissociation constant. $K_d$ is raised to the power $n$. This ensures that its dimension of concentration remains constant as $n$ varies. Equation (1) offers a particular understanding of biomechanical forces obtained from different dimensions of the sensing elements and the impact of mechanical connectivity networks on the ligand-receptor binding interactions and may help to design better assays for direct mechanical assays of ligands present at ultralow concentrations. So to quantify the reliance of signal reproducibility on the mechanical connectivity networks, the experimental data was modelled using equation (1) and the outcome of the fitted results, revealed a striking similarity in $K_d$ values of $0.5 \pm 0.1$ $\mu$M for 100 $\mu$m and 70 $\mu$m cantilevers over a wide dynamic range of ligand concentrations. To account for the consistent binding constants, the formulated hypothesis is that whereas the limits of biomechanical force sensitivity may be influenced by the size of the sensing element, the binding thermodynamics is unaffected. This is because it is a geometry-independent quantity which relies only on the interplay between biomechanical forces and the concentration of ligands in solution. Consequently, the binding constant of any specific ligand-receptor interaction is essentially identical whether it occurs in a three-dimensional (3D) solution or at 2D-surface receptors. The differences in the binding constant, therefore, could be as a result of the disparities in the biomechanical forces and from any other effects that may alter the signal reproducibility. To lend support to our hypothesis, a comparison of the binding analysis for Van with conventional methods such as the 2D-surface plasmon resonance (SPR) assay ($K_d$ of $1.0 \pm 0.3$ $\mu$M[4]) and 3D-solution assay ($K_d$ of 0.7 $\mu$M[41]) was made, even though these techniques do not respond to changes in biomechanical forces. The agreement of $K_d$ values across a variety of techniques and between different sizes of the sensing elements is a further confirmation that mechanical connectivity network is crucial in establishing reproducibility in mechanical signaling.

**Characterization of enantiomeric drugs using parallel array networks**

Finally, learning from enantiomeric molecules, although identical in structure and are mirror images of one another with different physical characteristics, could be used to test the impact of the regulatory connectivity networks on the biomechanical forces involved in molecular interactions. So it was inferred that in chiral species the level of complementarity between a drug and its target will lead to different assemblies across the mirror images of the surface receptors and this can be reflected in the distinguishable biomechanical forces. To test this concept the interaction of the D-VSR and its corresponding mirror image (L-VSR) (see, Cantilever functionalization with surface target molecules) with the antibiotic drug vancomycin was studied. D-VSR is an enantiomer predominantly found in bacterial cell walls whereas L-VSR is not produced by bacteria. This suggests that there may be some selective advantages of having only one type of enantiomer and this may be reflected by differences in the binding affinity of D-VSR and L-VSR with their ligands. The mechanical response obtained using a high concentration of Van (50 μM) against 100 μm wide parallel transduction arrays of D-VSR and L-VSR was initially tested. A large mechanical response of --4000 pN) was observed with the D-VSR coated cantilevers. In contrast the signal response of Van against L-VSR was very small ~-200 pN, approximately 20 times smaller than with D-VSR (Fig. 5a). The experiment was repeated using a wide range of different concentrations of Van (0.1 - 1000 μM) on at least four separate chips. The results from these experiments which are summarized in Fig. 5b demonstrate that as the concentration of Van increased, the compressive biomechanical force against D-VSR increased accordingly. A plateau was reached at a concentration of 50 μM Van. In contrast, only a small signal response for L-VSR for all concentrations of Van was observed.

The experiments were repeated using another clinically relevant molecule known as ristocetin (Rist). Rist is used for diagnosis of von Willebrand's disease by virtue of its ability to strongly bind a glycoprotein known as the von Willebrand's factor[42]. Figure 5c shows the signal response obtained when Rist was initially used at 50 μM concentration with both D-VSR and L-VSR coated cantilevers. The signal response generated from D-VSR --5000 pN was found to be approximately 3 times larger than that obtained from L-VSR --2000 pN. The experiment was repeated with an even wider range of differing Rist concentrations (0.001 to 1000 μM) on at least four separate chips. The signal response with L-VSR remained small compared to D-VSR even when high concentrations of Rist were employed. Generally, the results with Rist closely reflect those obtained with Van where a significatly large difference in mechanical response was observed between D-VSR and L-VSR(Fig. 5d). This shows for the first time that distinct biomechanical forces are generated by complementary enantiomeric drug molecules and their respective targets which can be accurately and reproducibly measured. The findings clearly demonstrate that chiral species of a drug molecule and its target generate distinct biomechanical forces, and so a further evidence for the effectiveness of mechanical connectivity networks on signal sensitivity and reproducibility. In particular, these findings are consistent with the idea that a naturally occuring antibiotic such as Van is able to specifically target the right-handed amino acid residues found in bacterial cell walls, both for enhanced biomechanical force transduction and microbial susceptibility[4].

**Cantilever fabrication and Au coating**

IBM Rushlikon 100 μm wide cantilevers were supplied by Concentris GmBH. The narrow 70 μm wide cantilevers were fabricated using standard microfabrication techniques on insulator (SOI) wafers. Cantilever arrays were cleaned by incubating them for 20 min in freshly prepared piranha solution, composed of 1:1 $H_2SO_4$ and $H_2O_2$, then rinsed thoroughly using ultrapure water followed by pure ethanol before being dried on a hotplate at 75 °C. Silicon cantilevers were coated with a 2 nm titanium film to act as an adhesion layer followed by a 20 nm layer of Au at a rate of 0.7 $nms^{-1}$ using an electron beam (BOC Edwards Auto 500, U.K., vacuum pressure of $10^{-7}$ mbar). Film thickness was monitored using a quartz crystal monitor placed directly above the target source. The freshly Au-coated chips were functionalized with surface target molecules using ethanolic thiol solutions.

**Cantilever binding assay**

Functionalized cantilever chips were mounted in a sealed liquid cell environment and a time-multiplexed optical laser readout system (Scentris, Veeco Instruments Inc., Santa Barbara, CA, USA) was used to measure the cantilever bending. The laser alignment was considered successful if the difference between the minimum and maximum deflection was < 5% when the cell temperature was raised by 1 °C for 10 min followed by 10 min cooling to room temperature. To ensure consistency between cantilevers, resonant frequency measurements were used to confirm that the variation in their spring constants was ≤ 1%.

The biomechanical force data from ligand-receptor interactions were measured as follows: sodium phosphate buffer (pH 7.4, 0.1 M) was injected into the cell for either 3 or 10 min to establish a baseline. Ligands in sodium phosphate buffer were then injected into the cell for 30-60 minutes. This was followed by a wash with sodium phosphate buffer (pH 7.4, 0.1 M) for 10 - 30 min and a subsequent wash with 10 mM HCI /or 10 mM glycine-HCI pH 2.5 for 30 min to dissociate the ligand receptor complex completely and regenerate the cantilever surface. A final wash in sodium phosphate buffer for 10 min was used to restore the baseline signal. Cantilever bending signals were measured in a fixed temperature-controlled hood at 25 °C using a liquid flow rate of 30-180 μL $min^{-1}$. This flow rate was chosen as it is known not to affect the absolute signal response of cantilevers.

**Cell culture**

Two different cell lines were used: the human breast cancer cell line MDA-MB231 and the human mammary epithelial cell line MCF10A (American Type Culture Collection, Manassas, VA). MDA-MB231 cell line was grown in DMEM medium, supplemented with 10% FBS, 1% Glutamine and 100 IU/ml Penicillin-Streptomycin and MCF10A cell line was cultured in a minimal essential growth medium (MEGM, Lonza, Cedarlane) supplemented with the same additives, where both cell lines were incubated at 37 °C in a 5% $CO_2$-95% $O_2$ incubator, and the growth media were exchanged every 48 h. To prepare the cells for mechanobiology, 100% confluent cells were detached from the culture flasks using manual cell scrapers without the use of trypsin to preserve cell structure and membrane proteins. They were centrifuged, diluted in serum-free media where an initial concentration of $10^3$ cells $ml^{-1}$ was re-suspended in PBS solution. The cells were initially aliquoted into a low-attachment 96-well plate and PBS solution used in the serial dilution steps until the required final concentration. The concentration of each cell line was determined by cell counting using an optical microscope. To determine the impact of microenvironment on the mechanical response of breast cancer cells, a mixture of cancer cells /normal mammary epithelial cells was prepared with a breast cancer cell molar fraction of 0.0, 0.01, 0.05, 0.25, 0.5, 0.75, 0.9 and 1.0 where the ratio of 0.0 indicates normal mammary epithelial cells and 1.0 represents solely of malignant breast cells. The MDA-MB231 and MCF10A were diluted in PBS solution to yield a total concentration of 1000 cells $ml^{-1}$.

**Mechanical connectivity in cell response**

**Quantitation of mechanical force from cell response**

Human epithelial cells (MDA- MB231) from malignant tumours or the noncancerous cells (MCF10A) were injected into the sealed liquid chamber at a flow rate of 1 ml $h^{-1}$. The mechanical force detection was based on monitoring the changes in the cantilever bending response caused by cell adhesion. Accordingly, a series of binding analysis of cell response was performed.

The mechanical response was recorded when the malignant cancer cells were attached to the peptide ligands grafted at the hinge area where the cell concentration was fixed at 25 cell $ml^{-1}$. Analysis of the nanomechanical forces exerted by malignant cancer cells revealed compressive force of -3200 $\pm$ 40 pN. The mechanical response of the cantilever using a similar concentration of malignant cancer cells but attached at the free-end was recorded. The mechanical response at the free-end is considered negligible when compared with the signals obtained at the hinge area (Fig. 9a). The widely differing nanomechanical signals obtained from the free-end and hinge region suggest that a mass effect alone is not sufficient to mediate considerable mechanical changes. Consequently, the signal is in part a function of the specific connectivity with the hinge and the interactions between the hinge area and free-end, the latter being a potential source of signal variability. To apply this phenomenon as a universal tool for rapid and ultrasensitive detection of nanomechanical interactions between cells and the substrate, the response in mechanobiology of cell signaling on microscale and biomolecular interactions in a series of SAM samples grafted continuously from hinge for varying geometric lengths of the regions covered by receptors (Fig. 3) was compared. The agreement of cantilever data between cells and molecules demonstrates convincingly that the hinge region is extremely sensitive to the changes in stress than the free-end and, makes this approach highly suitable for the fabrication of ultrasensitive sensors for label-free amplification of a signal generated by analytes at low concentrations. As a further measurement control and to test the specificity of the hinge in the mediation of signaling in response to stress, a non-neoplastic epithelial cells at 25 cell $ml^{-1}$ to match the experimental conditions for the malignant cancer cells was used. The signals from the hinge area and full cantilever simultaneously were reordered. The analysis revealed negligible mechanical response for both the hinge area and full cantilever compared with those achieved for the cancer cells (Fig. 9a,b), thereby confirming that the observed signal in response to the malignant cancer cells is, to a large extent, a preserve of the hinge area.

The spatial connectivity network on the mechanotransduction and propagation of forces at the hinge area and full cantilever was further investigated. Using a total concentration of malignant cancer cells initially kept constant at 25 cell $ml^{-1}$, a relatively large signal from the hinge area in contrast to the full cantilever (Fig. 9b and 10) was observed.

In Figure 10, it is clearly shown that the cancer targeting peptides preferentially bind to breast cancer cells compared to noncancerous epithelial cells. It was found that the geometrical lengths of the regions covered by receptors and their location play very significant role in sensitivity of the mechanical force generated by ligand-receptor binding interactions. This is surprising, given a fully covered cantilever is expected to have a higher number of peptide ligands and therefore a large mechanical response. The large signal response in case of the hinge area is because a relatively large fraction of the surface is covered, resulting into high levels of mechanical connectivity. Consequently, the increased level of connectivity between chemically transformed regions of the surface, favours the short-range repulsive interactions such as steric interactions between the nodes of the nanomechanical network to propagate the large mechanical forces exerted by cell attachment. Notably, these measurements reveal a key insight into the signaling pathways to stress and how the signal is relayed in living cells.

To test further the mechanisms of action of cell signaling, a higher cell concentration at 500 cell $ml^{-1}$ was chosen. This particular concentration gives a saturation signal, because most accessible peptide-binding sites are occupied and the attached cancer cells are expected to be closest to each other. Fig. 9b shows the outcome where the full cantilever surface improves the apparent mechanical force 2-fold and, compared to the hinge area, confirms that the enhanced nanomechanical forces exerted by cancer cells is strongly linked to the connectivity of chemically transformed regions. Thus, the results show that for a large mechanical response in cancer cells to be accomplished, both the selectivity of cells to surface targets and connectivity with the hinge region are important key parameters.

Next, the underlying effects of microenvironments on the signaling of cancer cells was analyzed. Direct mechanical assays in which cancer cells were mixed with non-neoplastic human mammary epithelial cells with defined ratio of ~10:990, 50:950 and 500:500, where the total cell concentration was fixed at 1000 cell ml-1 were performed. Control experiments performed in the absence of competing epithelial cells, resulted in cell response with signals approximately equal in magnitude, for each ratio of cancer cells to epithelial cells. Therefore, these experiments unequivocally demonstrate that the mechanical signals observed in a mixture of cells is in fact related to biospecific recognition between cancer cells and the homing peptides. The relatively large mechanical signals in the mixture could be because of the high levels of receptor expressions[41,42] on the surface of cancer cells. The overexpressed receptors in cancer cells compared to normal cells could act as the driving force to create a strong interaction with the surface targets, for example to enable communication between specific neighbouring cancer cells while ignoring signals from the epithelial cells.

To quantitatively determine how the exerted mechanical force is influenced by effects such as cell population as well as the physical location of chemically reacted regions, the signals in cell response at the hinge region and full cantilever were measured by varying the concentration of cancer cells in solution. It was found that the generated force was slightly smaller at lower cancer cell population but steeply increased as a function of concentration followed by saturation, when most accessible surface binding sites were occupied (Fig. 9d-f). The maximum nanomechanical force generated by malignant cells was -9000 ± 50 pN for the hinge area and -10,000 ± 80 pN for the full cantilever. The findings further provide mechanistic insights into how cells interact with one another and could further improve our understanding of how these interactions enable cancer cells to communicate with one another and with their microenvironments, enabling them to invade the surrounding tissues and how as clusters they are able to coordinate their migration through narrow blood capillaries[43].

**Biomechanical force analysis**

The analysis of mechanical force (F) arising from the binding of a ligand to its receptor was quantified using the equation

$$F = k\Delta z$$

(2)

Here k corresponds to the cantilever's nominal spring constant and $\Delta z$ is the differential mechanical deflection signal. The nominal spring constant for broad IBM fabricated silicon cantilever arrays (1 μm thick, 500 μm long and 100 μm wide) is 0.02 $Nm^{-1}$ whereas for narrow fabricated silicon cantilever arrays (1 μm thick, 500 μm long and 70 μm wide), the spring constant is 0.014 $Nm^{-1}$ if the linear scaling is assumed. For simplicity, the biomechanical force analysis of the data for both geometries of cantilever arrays was performed with $k$ = 0.02 $Nm^{-1}$. The differential equilibrium mechanical force ($F_{\mathrm{diff}}$) was calculated by subtracting the *in-situ* reference mechanical response $\Delta F_{ref}$ from the absolute mechanical response $\Delta F_{abs}$. The entire set of differential equilibrium biomechanical forces for a wide range of concentrations of ligands (0.0005 - 1000 μM) were determined using 4 chips for each ligand (each chip containing 8 cantilevers). For each ligand concentration, the arithmetic mean of the differential equilibrium biomechanical force data ($\Delta F_{\mathrm{eq}}$) across 4 chips was calculated using the equation

$$\Delta F_{eq} = \frac{\sum F_{diff}}{n}$$

(3)

Here $n$ is the number of measurements. The standard deviation of the biomechanical force data ($\sigma$) was calculated using the equation

$$\sigma = \sqrt{\frac{\left(\Delta F_{eq} - \Delta F_{diff}\right)}{n-1}}$$

(4)

The standard error (SE) of the differential equilibrium biomechanical force for each ligand concentration was calculated using the equation

$$SE = \frac{\sigma}{\sqrt{n}}$$

(5)

**Quantitation of biomechanical force of ligand-receptor binding interactions**

In this model, the surface receptor (R) was considered to react reversibly with a ligand *(ligand)* to form ligand-receptor complex *(ligand.R)* on the surface. The reactions are then quantified by considering the expression

$$n(ligand) + R \underset{K_{off}}{\overset{K_{on}}{\rightleftharpoons}} \left((ligand)_n.R\right)$$

(6)

Here *n* is the stoichiometric coefficient of the reaction. The expression correlating the concentrations between a ligand in solution and surface receptor is

$$K_d^n\left[\left((ligand)_n.R\right)\right] = [ligand]_{free}^n[R]$$

(7)

Here $K_d$ is the surface thermodynamic equilibrium dissociation constant. The total concentration of the surface receptor $[R]_T$ is

$$[R]_T = [R]_{free} + \left[(ligand)_n.R\right]$$

(8)

Using equations (7) and (8), the total amount of a ligand bound at the surface is determined using the expression

$$\theta = \frac{[ligand]^n}{K_d^n + [ligand]^n}$$

(9)

Here $\theta = [(ligand_1)_n.R]/[R]_T$ is the fraction of the surface occupied by the binding sites. If it is assumed that the biomechanical force (F) involved in ligand-receptor complex interactions scales in direct proportion to the surface coverage $(F = F_{max}\theta)$, then expression (9) is adjusted accordingly to give equation (1).

$$F_{eq} = \frac{F_{max}[ligand]^n}{K_d^n + [ligand]^n} \quad (10)$$

**Reference**

(1) Son, K., Guasto, J. S. & Stocker, R. Bacteria can exploit a flagellar buckling instability to change direction. Nat. Phys. 9, 494-498 (2013).

(2) Lele, P. P., Hosu, B. G. & Berg, H. C. Dynamics of mechanosensing in the bacterial flagellar motor. Proc. Natl. Acad. Sci. USA, 110, 11839-11844 (2013).

(3) Gebhardt, J. C. & Rief, M. Biochemistry force signaling in biology. Science 324, 1278-1280 (2009).

(4) Ndieyira, J. W. et al. Surface mediated cooperative interactions of drugs enhance mechanical forces for antibiotic action. Sci. Rep. 7, 41206; doi: 10.1038/srep41206 (2017).

(5) Wu, G. H. et al. Bioassay of prostate-specific antigen (PSA) using microcantilevers. Nat. Biotechnol. 19, 856-860 (2001).

(6) Fritz, J. et al. Translating biomolecular recognition into nanomechanics. Science 288, 316-318 (2000).

(7) Berger, R. et al. Surface-stress in the self-assembly of alkanethiols on gold. Science. 276, 2021-2024 (1997).

(8) Wirtz, D., Konstantopoulos, K. & Searson, P. C. The physics of cancer: the role of physical interactions and mechanical forces in metastasis. Nat. Rev. Cancer. 11, 512-522 (2011).

(9) Hoffman, B. D., Grashoff, C. & Schwartz, M. A. Dynamic molecular processes mediate cellular mechanotransduction. Nature 475, 316-323 (2011).

(10) Dutta, P. et al. Enantioselective sensors based on antibody-mediated nanomechanics. Anal. Chem. 75, 2342-2348 (2003).

(11) Ndieyira, J. W. et al. Nanomechanical detection of antibiotic-mucopeptide binding in a model for superbug drug resistance. Nat. Nanotechnol. 3, 691-696 (2008).

(12) Huber, F., Lang, H. P., Backmann, N., Rimoldi, D. & Gerber, Ch. Direct detection of a BRAF mutation in total RNA from melanoma cells using cantilever arrays. Nat. Nanotechnol. 8, 125-129 (2013).

(13) Etayash, H., Khan, M. F., Kaur, K. & Thundat, T. Microfluidic cantilever detects bacteria and measures their susceptibility to antibiotics in small confined volumes. Nat. Commun. 7, 12947 (2016).

(14) Kosaka, P. M. et al. Detection of cancer biomarkers in serum using a hybrid mechanical and optoplasmonic nanosensor. Nat. Nanotechnol. 9, 1047-1053 (2014).

(15) Long, G. et al. Rapid detection of bacterial resistance to antibiotics using AFM cantilevers as nanomechanical sensors. Nat. Nanotechnol. 8, 522-526 (2013).

(16) Braun, T. et al. Quantitative time resolved measurements of membrane protein-ligand interactions using microcantilever arrays sensors. Nat. Nanotechnol. 4, 179-185 (2009).

(17) Shekhawat, G., Tark, S-H. & Dravid, V. P. MOSFET-Embedded microcantilevers for measuring deflection in biomolecular sensors. Science 311, 1592-1595 (2006).

(18) Johansson, A., Blagoi, G. & Boisen, A. Polymeric-based biosensors with integrated readout. Appl. Phys. Lett. 89, 173505 (2006).

(19) Lavrik, N. V., Sepaniak, M. J. & Datskos, P. G. Cantilever transducers as a platform for chemical and biological sensors. Rev. Sci. Instrum. 75, 2229-2253 (2004).

(20) Raiteri, R., Grattarola, M., Butt, H. J. & Skl'adal, P. Micromechanical cantilever-based biosensors Sensors Actuators. Sensors Actuators B79, 115-126 (2001).

(21) Ndieyira, J. W. et al. Surface-stress sensors for rapid and ultrasensitive detection of active free drugs in human serum. Nat. Nanotechnol. 9, 225-232 (2014).

(22) Patil, S. B. et al. Decoupling competing surface binding kinetics and reconfiguration of receptor footprint for ultrasensitive stress assays. Nat. Nanotechnol. 10, 899-907 (2015).

(23) Malvankar, N. S., Yalcin, S. E., Tuominen, M. T. & Lovley, D. R. Visualization of charge propagation along individual pili proteins using ambient electrostatic force microscopy. Nat. Nanotechnol. 9, 1012-1017 (2014).

(24) Tetard, L., Passian, A., Farahi, R. H., Thundat, T. & Davison, B. H. Opto-nanomechanical spectroscopic material characterization. Nat. Nanotechnol. 10, 870-877 (2015).

(25) Dupres, V. et al. Nanoscale mapping and functional analysis of individual adhesins on living bacteria. Nat. Meth. 2, 515-520 (2005).

(26) Dupres, V. Using nanotechniques to explore microbial surfaces. Nat. Rev. Microbiol. 2, 451-460 (2004).

(27) Zhang, J. et al. Rapid and label-free nanomechanical detection of biomarker transcripts in human RNA. Nat. Nanotechnol. 1, 214-220 (2006).
(28) Backmann, N. et al. A label-free immunosensor array using single-chain antibody fragments. Proc. Natl. Acad. Sci. USA, 102, 14587-14592 (2005).
(29) Mukhopadhyay, R. et al. Cantilever sensor for nanomechanical detection of specific protein conformations. Nano Lett. 5, 2385-2388 (2005).
(30) Garcia, R., Knoll, A. W. & Riedo, E. Advanced scanning probe lithography. Nat. Nanotechnol. 9, 577-587 (2014).
(31) Lee, K. B., Park, S. J., Mirkin, C. A., Smith, J. C. & Mrksich, M. Protein nanoarrays generated by dip-pen nanolithography. Science 295, 1702-1705 (2002).
(32) Salaita, K., Wang, Y. & Mirkin, C. A. Applications of dip-pen nanolithography. Nat. Nanotechnol. 2, 145-155 (2007).
(33) Love, J. C., Estroff, L. A., Kriebel, J. K., Nuzzo, R. G. & Whitesides, G. M. Self-assembled monolayers of thiolates on metals as a Form of nanotechnology. Chem. Rev. 105, 1103-1169 (2005).
(34) Cho Y. R., Entress R. M. & Williams, D. H. Synthesis of cell-wall analogues of vancomycin-resistant enterococci using solid phase peptide synthesis. Tetrahedron Lett., 38, 5229-5232 (1997).
(35) Kaufman, E. D. et al. Probing protein adsorption onto mercaptoundecanoic acid stabilized gold nanoparticles and surfaces by quartz crystal microbalance and zeta-potential measurements. Langmuir 23, 6053-6062 (2007).
(36) Watari, M. et al. Investigating the molecular mechanisms of in-plane mechanochemistry on cantilever arrays. J. Am. Chem. Soc.129, 601-607 (2007).
(37) Stoney, G. G. The tension of metallic films deposited by electrolysis. Proc. Roy. Soc. London, Ser. A. 82, 172-175 (1909).
(38) Jarvis, W. R. Epidemiology, appropriateness, and cost of vancomycin use. Clin. Infect. Dis. 26, 1200-1203 (1998).
(39) Sieradzki, K. et al. The development of vancomycin resistance in a patient with methicillin-resistant Staphylococcus aureus infection. NEJM. 340, 517-523 (1999).
(40) Burg, T. P. et al. Weighing of biomolecules, single cells and single nanoparticles in fluid. Nature 446, 1066-1069 (2007).
(41) Nieto, M. & Perkins, H. R. Modifications of the Acyl-D-alanyl-D-alanine terminus affecting complex-formation with vancomycin. Biochem. J. 123, 789-803 (1971).
(42) Rodeghiero, F., Castaman, G. & Dini, E. Epidemiological investigation of the prevalence of von Willebrand's disease. Blood 69, 454-459 (1987).
(43) Ahmed, S., Mathews, A. S., Byeon, N., Lavasanifar, A. & Kaur, K. Peptide arrays for screening cancer specific peptides. Anal. Chem. 82, 7533-7541 (2010).

## Claims

**1.** A cantilever sensor comprising:

a silicon layer having at least two surfaces and at least two end regions, wherein at least one surface is coated with a coating comprising Au, and one end region is anchored to a support, thereby forming a hinge region between the silicon layer and the support,
wherein the at least one Au-coated surface is further coated with a self-assembled monolayer network of probe molecules that covers between 30% and 80% of the Au-coated layer surface and is arranged along the longitudinal length of the cantilever or transverse to the long axis of the cantilever in a continuous connectivity between the probe molecules of the network and between the network and the hinge region of the cantilever, wherein said continuous connectivity is obtained when the distance between a self-assembled monolayer network of probe molecules and a hinge region of the cantilever is equal or less than 50 μm and when the distance between the plurality of probe molecules is equal or less than 50 μm.

**2.** The cantilever according to claim 1, wherein the self- assembled monolayer network of probe molecules covers between 30 and 70%, or between 30 and 60% or between 30 and 50%, or between 30 and 40% of the Au layer surface.

**3.** The cantilever according to claim 1 or 2, wherein the surface opposite to the Au-coated surface is unpassivated or said surface is passivated with a PEG-silane coating.

**4.** The cantilever according to any claim 1 to 3, wherein at least one surface is coated with a first layer of titanium on top of which the Au layer is deposited.

**5.** The cantilever according to any claim 1 to 4, having a width of equal or less than 100 μm.

**6.** An array comprising at least 8 unpassivated cantilever sensors according to any claim 1 to 5.

**7.** A method for detecting the presence of a coupling molecule in an isolated body fluid, comprising the steps of:

1) providing a cantilever sensor according to any claim 1 to 6;
2) contacting the cantilever sensor with an isolated body fluid containing the coupling molecule to be detected;
3) detecting the response signal due to cantilever bending; and
4) correlating the response signal to the presence or absence of the coupling molecule to be detected.

**8.** The method according to claim 7, wherein the body fluid is blood, plasma, saliva, sputum, or urine.

**9.** A process for the preparation of the cantilever according to any one of claims 1 to 5, comprising the steps of:

(i) impregnating a stamp with a solution of self-assembled monolayers (SAMs) of probe molecules by incubating for 1-5 minutes, preferably 1-2 minutes;
(ii) removing excess SAMs solution from the stamp by blowing an inert gas over the stamp;
(iii) placing the stamp in contact with the Au-coated surface of the cantilever for 1-5 minutes by applying a pressure to let the SAMs diffuse from the stamp onto the substrate thus enabling uniform molecular printing;
(iv) removing the stamp after 1-5 minutes.

**10.** The process of claim 9 comprising a further step of blocking the un-patterned areas on the Au-coated surface with non-specific SAMs which do not couple with a ligand.

**11.** A process for the preparation of the cantilever according to any one of claims 1 to 5, comprising the steps of:

(i) transferring a solution of self-assembled monolayers (SAMs) of probe molecules using a sharp scanning cantilever tip loaded the solution onto the Au-coated surface;
(ii) while transferring the solution, tracing a patterns of SAMs of probe molecules.

**12.** The process according to claim 11 comprising a further step of blocking the un-patterned areas on the Au-coated surface with non-specific SAMs which do not couple with a ligand.

**13.** The process according to claim 9 or 11, in which if the cantilever is unpassivated a further step of applying a PEG-silane coating on the side of the cantilever which is opposite to the Au-coated surface.

## Patentansprüche

**1.** Auslegersensor, umfassend:

eine Siliziumschicht, die mindestens zwei Oberflächen und mindestens zwei Endregionen aufweist, wobei mindestens eine Oberfläche mit einer Beschichtung beschichtet ist, die Au umfasst, und eine Endregion an einem Träger verankert ist, wodurch eine Gelenkregion zwischen der Siliziumschicht und dem Träger gebildet wird, wobei die mindestens eine Au-beschichtete Oberfläche ferner mit einem selbstorganisierten Monoschichtnetzwerk von Sondenmolekülen beschichtet ist, das zwischen 30 % und 80 % der Oberfläche der Au-beschichteten Schicht bedeckt und entlang der Längserstreckung des Auslegers oder quer zur Längsachse des Auslegers in einer kontinuierlichen Verbindung zwischen den Sondenmolekülen des Netzwerks und zwischen dem Netzwerk und der Gelenkregion des Auslegers angeordnet ist, wobei die kontinuierliche Verbindung erhalten wird, wenn der Abstand zwischen einem selbstorganisierten Monoschichtnetzwerk von Sondenmolekülen und einer Gelenkregion des Auslegers gleich oder kleiner als 50 μm ist und wenn der Abstand zwischen der Vielzahl von Sondenmolekülen gleich oder kleiner als 50 μm ist.

**2.** Ausleger nach Anspruch 1, wobei das selbstorganisierte Monoschichtnetzwerk von Sondenmolekülen zwischen 30 und 70 % oder zwischen 30 und 60 % oder zwischen 30 und 50 % oder zwischen 30 und 40 % der Au-Schicht-Oberfläche bedeckt.

**3.** Ausleger nach Anspruch 1 oder 2, wobei die Oberfläche, die der Au-beschichteten Oberfläche gegenüberliegt, nicht passiviert ist oder die Oberfläche mit einer PEG-Silan-Beschichtung passiviert ist.

**4.** Ausleger nach einem der Ansprüche 1 bis 3, wobei mindestens eine Oberfläche mit einer ersten Schicht aus Titan beschichtet ist, auf der die Au-Schicht abgeschieden ist.

**5.** Ausleger nach einem der Ansprüche 1 bis 4 aufweisend eine Breite gleich oder weniger als 100 $\mu$m.

**6.** Anordnung umfassend mindestens 8 nicht passivierte Auslegersensoren nach einem der Ansprüche 1 bis 5.

**7.** Verfahren zum Detektieren der Anwesenheit eines Kopplungsmoleküls in einer isolierten Körperflüssigkeit, umfassend die Schritte:

1) Bereitstellen eines Auslegersensors nach einem der Ansprüche 1 bis 6;
2) Inkontaktbringen des Auslegersensors mit einer isolierten Körperflüssigkeit, die das zu detektierende Kopplungsmolekül enthält;
3) Detektieren des Antwortsignals aufgrund von Auslegerbiegung; und
4) Korrelieren des Antwortsignals mit der Anwesenheit oder Abwesenheit des zu detektierenden Kopplungsmoleküls.

**8.** Verfahren nach Anspruch 7, wobei es sich bei der Körperflüssigkeit um Blut, Plasma, Speichel, Sputum oder Urin handelt.

**9.** Verfahren zur Herstellung des Auslegers nach einem der Ansprüche 1 bis 5, umfassend die Schritte:

(i) Imprägnieren eines Stempels mit einer Lösung von selbstorganisierten Monoschichten (SAMs) von Sondenmolekülen durch 1-5-minütiges Inkubieren, vorzugsweise 1-2-minütiges Inkubieren;
(ii) Entfernen der überschüssigen SAMs-Lösung aus dem Stempel, indem ein Inertgas über den Stempel geblasen wird;
(iii) Inkontaktbringen des Stempels mit der Au-beschichteten Oberfläche des Auslegers für 1-5 Minuten durch Ausüben eines Drucks, um die SAMs vom Stempel auf das Substrat diffundieren zu lassen, wodurch ein einheitlicher molekularer Druck ermöglicht wird;
(iv) Entfernen des Stempels nach 1-5 Minuten.

**10.** Verfahren nach Anspruch 9, umfassend einen weiteren Schritt zum Blockieren der unstrukturierten Bereiche auf der Au-beschichteten Oberfläche mit unspezifischen SAMs, die nicht mit einem Liganden koppeln.

**11.** Verfahren zur Herstellung des Auslegers nach einem der Ansprüche 1 bis 5, umfassend die Schritte:

(i) Übertragen einer Lösung von selbstorganisierten Monoschichten (SAMs) von Sondenmolekülen unter Verwendung einer scharf abtastenden Auslegerspitze, die die Lösung auf die Au-beschichtete Oberfläche lädt;
(ii) bei der Übertragung der Lösung, Nachverfolgen eines Musters von SAMs von Sondenmolekülen.

**12.** Verfahren nach Anspruch 11, umfassend einen weiteren Schritt zum Blockieren der unstrukturierten Bereiche auf der Au-beschichteten Oberfläche mit unspezifischen SAMs, die nicht mit einem Liganden koppeln.

**13.** Verfahren nach Anspruch 9 oder 11, wobei, wenn der Ausleger nicht passiviert ist, ein weiterer Schritt zum Aufbringen einer PEG-Silan-Beschichtung auf die Seite des Auslegers, die der Au-beschichteten Oberfläche gegenüberliegt, vorgesehen ist.

**Revendications**

**1.** Capteur cantilever, comprenant :

une couche de silicium comportant au moins deux surfaces et au moins deux régions d'extrémité, dans lequel au moins une surface est revêtue d'un revêtement comprenant de l'Au, et une région d'extrémité est ancrée à un support, formant ainsi une région charnière entre la couche de silicium et le support,

dans lequel l'au moins une surface revêtue d'Au est de plus revêtue d'un réseau monocouche autoassemblé de molécules sondes qui couvre entre 30 et 80 % de la surface de la couche revêtue d'Au et qui est disposé le long de la longueur longitudinale du cantilever ou transversalement à l'axe long du cantilever dans une connectivité continue entre les molécules sondes du réseau et entre le réseau et la région charnière du cantilever, dans lequel ladite connectivité continue est obtenue lorsque la distance entre un réseau monocouche autoassemblé de molécules sondes et une région charnière du cantilever est égale ou inférieure à 50 $\mu$m et lorsque la distance entre la pluralité de molécules sondes est égale ou inférieure à 50 $\mu$m.

2. Cantilever selon la revendication 1, dans lequel le réseau monocouche autoassemblé de molécules sondes couvre entre 30 et 70 %, ou entre 30 et 60 % ou entre 30 et 50 %, ou entre 30 et 40 % de la surface de la couche Au.

3. Cantilever selon la revendication 1 ou 2, dans lequel la surface opposée à la surface recouverte d'Au est non passivée ou ladite surface est passivée avec un revêtement de PEG-silane.

4. Cantilever selon l'une quelconque des revendications 1 à 3, dans lequel au moins une surface est revêtue d'une première couche de titane sur laquelle est déposée la couche Au.

5. Cantilever selon l'une quelconque des revendications 1 à 4, ayant une largeur inférieure ou égale à 100 $\mu$m.

6. Réseau comprenant au moins 8 capteurs cantilevers non passivés selon l'une quelconque des revendications 1 à 5.

7. Procédé pour détecter la présence d'une molécule de couplage dans un fluide corporel isolé, comprenant les étapes de :

1) fournir un capteur cantilever selon l'une quelconque des revendications 1 à 6 ;
2) mettre en contact le capteur cantilever avec un fluide corporel isolé contenant la molécule de couplage à détecter ;
3) détecter le signal de réponse dû à la flexion du cantilever ; et
4) corréler le signal de réponse à la présence ou à l'absence de la molécule de couplage à détecter.

8. Procédé selon la revendication 7, dans lequel le fluide corporel est du sang, du plasma, de la salive, du crachat ou de l'urine.

9. Procédé de préparation du cantilever selon l'une quelconque des revendications 1 à 5, comprenant les étapes de :

(i) imprégner un tampon avec une solution de monocouches autoassemblées (SAMs) de molécules sondes par incubation pendant 1-5 minutes, de préférence 1-2 minutes ;
(ii) éliminer l'excès de solution de SAMs du tampon en soufflant un gaz inerte sur le tampon ;
(iii) placer le tampon en contact avec la surface revêtue d'Au du cantilever pendant 1-5 minutes en appliquant une pression pour laisser les SAMs se diffuser du tampon sur le substrat, permettant ainsi une impression moléculaire uniforme ;
(iv) retirer le tampon après 1-5 minutes.

10. Procédé selon la revendication 9, comprenant une étape supplémentaire consistant à bloquer les zones sans motif sur la surface revêtue d'Au avec des SAMs non spécifiques qui ne se couplent pas avec un ligand.

11. Procédé de préparation du cantilever selon l'une quelconque des revendications 1 à 5, comprenant les étapes de :

(i) transférer une solution de monocouches autoassemblées (SAMs) de molécules sondes en utilisant une pointe de cantilever de balayage net chargeant de la solution sur la surface revêtue d'Au ;
(ii) tout en transférant la solution, tracer un motif de SAMs de molécules sondes.

12. Procédé selon la revendication 11, comprenant une étape supplémentaire consistant à bloquer les zones sans motif sur la surface revêtue d'Au avec des SAMs non spécifiques qui ne se couplent pas avec un ligand.

13. Procédé selon la revendication 9 ou 11, dans lequel, si le cantilever n'est pas passivé, une étape supplémentaire consiste à appliquer un revêtement de PEG-silane sur le côté du cantilever qui est opposé à la surface revêtue d'Au.

Fig. 1

a)

b)

c)

d)

e)

f)

a)
b)
reference
parallel arrays
Biomechanical force (nN)
Differential bending signal (nm)
Time (min)
c)
d)
reference
transverse arrays
Biomechanical force (nN)
Differential bending signal (nm)
Time (min)
e)
f)
reference
parallel arrays
Biomechanical force (nN)
Differential bending signal (nm)
Time (min)
g)
h)
reference
parallel arrays
Biomechanical force (nN)
Differential bending signal (nm)
Time (min)

Fig. 2

Fig. 3

a)

b)

reference
free-end
hinge region
Biomechanical force (nN)
Differential bending signal (nm)
Time (min)
1
0
-1
-2
-3
-4
50
0
-50
-100
-150
-200
0
3
6
9
12

c)

d)

hinge region
Biomechanical force (nN)
Differential bending signal (nm)
Patching length from the hinge region (µM)
0.0
-0.2
-0.4
-0.6
-0.8
-1.0
0
-10
-20
-30
-40
-50
0
100
200
300

e)

f)

free end
Biomechanical force (nN)
Differential bending signal (nm)
Patching length from the free end (µM)
0.2
0.0
-0.2
-0.4
-0.6
-0.8
-1.0
10
0
-10
-20
-30
-40
-50
0
100
200
300

Fig. 4

a)
Van (500 pM)
Biomechanical force (nN)
Differential bending signal (nm)
Time (min)
b)
PEG
20 nM
50 nM
500 nM
Biomechanical force (nN)
Differential bending signal (nm)
Time (min)
c)
1 μM Van
10 mM HCl
Biomechanical force (nN)
Differential bending signal (nm)
Time (min)
d)
1 μM Van
PEG
Broad cantilever
Narrow cantilever
Biomechanical force (nN)
Differential bending signal (nm)
Time (min)
e)
Broad cantilever
Narrow cantilever
Biomechanical force (nN)
Differential bending signal (nm)
[Van] (μM)
f)
100 fg/ml
5 fg/ml
0.2 fg/ml
PEG
Biomechanical force (nN)
Differential bending signal (nm)
Time (min)

Fig. 5

a)
b)
c)
d)
Biomechanical force (nN)
Differential bending signal (nm)
Time (min)
[Van] (M)
[Rist] (M)
PEG
L-VSR
D-VSR

Fig. 6

a)
b)
c)
d)

Fig. 7

a)

b)

Fig. 8

a)

b)

c)

d)

**FIG.9a-b**

a)
A C Hinge area
B D Free-end
B
D
C
A
Mechanical force (nN)
Differential bending signal (nm)
8
4
0
-4
-8
-12
400
200
0
-200
-400
-600
0
3
6
9
12
15
18
21
Time (min)

b)
C A Hinge area
D B E Full cantilever
B
E
A
C
D
Mechanical force (nN)
Differential bending signal (nm)
8
4
0
-4
-8
-12
400
200
0
-200
-400
-600
0
3
6
9
12
15
18
21
Time (min)

FIG.9c-d

c)

Control
A 10 900 cells mL$^{-1}$
B 50 950 cells mL$^{-1}$
C 500 500 cells mL$^{-1}$
Mechanical force (nN)
Differential bending signal (nm)
Time (min)
8
4
0
-4
-8
-12
400
200
0
-200
-400
-600
0
3
6
9
12
15
A
B
C

d)

Control
A 10 cells mL$^{-1}$
B 50 cells mL$^{-1}$
C 500 cells mL$^{-1}$
Mechanical force (nN)
Differential bending signal (nm)
Time (min)
8
4
0
-4
-8
-12
400
200
0
-200
-400
-600
0
3
6
9
12
15
18
21
A
B
C

FIG.9e-f

e)

Control
A 10 cells mL$^{-1}$
B 50 cells mL$^{-1}$
C 500 cells mL$^{-1}$
Mechanical force (nN)
Differential bending signal (nm)
Time (min)
A
B
C

f)

A Full cantilever
B Hinge area
Equation (1), $R^2$ = 0.99
Mechanical force (nN)
Differential bending signal (nm)
Cancer cells (cell mL$^{-1}$)
A
B

FIG.10

Hinge vs. cancer cells A
Whole vs. cancer cells B
Free-end vs. cancer C
Hinge vs. normal cells D
Whole vs. normal cells E
Free-end vs. normal cells F
Buffer
A
B
C, D, E
F
Biomechanical force (nN)
Differential binding signal (nm)
Time (min)
Hinge
Whole
Free-end

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SON, K. ; GUASTO, J. S. ; STOCKER, R.** Bacteria can exploit a flagellar buckling instability to change direction. *Nat. Phys.,* 2013, vol. 9, 494-498
- **LELE, P. P. ; HOSU, B. G. ; BERG, H. C.** Dynamics of mechanosensing in the bacterial flagellar motor. *Proc. Natl. Acad. Sci. USA,* 2013, vol. 110, 11839-11844
- **GEBHARDT, J. C. ; RIEF, M.** Biochemistry force signaling in biology. *Science,* 2009, vol. 324, 1278-1280
- **NDIEYIRA, J. W. et al.** Surface mediated cooperative interactions of drugs enhance mechanical forces for antibiotic action. *Sci. Rep.,* 2017, vol. 7, 41206
- **WU, G. H. et al.** Bioassay of prostate-specific antigen (PSA) using microcantilevers. *Nat. Biotechnol.,* 2001, vol. 19, 856-860
- **FRITZ, J. et al.** Translating biomolecular recognition into nanomechanics. *Science,* 2000, vol. 288, 316-318
- **BERGER, R. et al.** Surface-stress in the self-assembly of alkanethiols on gold. *Science,* 1997, vol. 276, 2021-2024
- **WIRTZ, D. ; KONSTANTOPOULOS, K. ; SEARSON, P. C.** The physics of cancer: the role of physical interactions and mechanical forces in metastasis. *Nat. Rev. Cancer.,* 2011, vol. 11, 512-522
- **HOFFMAN, B. D. ; GRASHOFF, C. ; SCHWARTZ, M. A.** Dynamic molecular processes mediate cellular mechanotransduction. *Nature,* 2011, vol. 475, 316-323
- **DUTTA, P. et al.** Enantioselective sensors based on antibody-mediated nanomechanics. *Anal. Chem.,* 2003, vol. 75, 2342-2348
- **NDIEYIRA, J. W. et al.** Nanomechanical detection of antibiotic-mucopeptide binding in a model for superbug drug resistance. *Nat. Nanotechnol.,* 2008, vol. 3, 691-696
- **HUBER, F. ; LANG, H. P. ; BACKMANN, N. ; RIMOLDI, D. ; GERBER, CH.** Direct detection of a BRAF mutation in total RNA from melanoma cells using cantilever arrays. *Nat. Nanotechnol.,* 2013, vol. 8, 125-129
- **ETAYASH, H. ; KHAN, M. F. ; KAUR, K. ; THUNDAT, T.** Microfluidic cantilever detects bacteria and measures their susceptibility to antibiotics in small confined volumes. *Nat. Commun.,* 2016, vol. 7, 12947
- **KOSAKA, P. M. et al.** Detection of cancer biomarkers in serum using a hybrid mechanical and optoplasmonic nanosensor. *Nat. Nanotechnol.,* 2014, vol. 9, 1047-1053
- **LONG, G. et al.** Rapid detection of bacterial resistance to antibiotics using AFM cantilevers as nanomechanical sensors. *Nat. Nanotechnol.,* 2013, vol. 8, 522-526
- **BRAUN, T. et al.** Quantitative time resolved measurements of membrane protein-ligand interactions using microcantilever arrays sensors. *Nat. Nanotechnol.,* 2009, vol. 4, 179-185
- **SHEKHAWAT, G. ; TARK, S-H. ; DRAVID, V. P.** MOSFET-Embedded microcantilevers for measuring deflection in biomolecular sensors. *Science,* 2006, vol. 311, 1592-1595
- **JOHANSSON, A. ; BLAGOI, G. ; BOISEN, A.** Polymeric-based biosensors with integrated readout. *Appl. Phys. Lett.,* 2006, vol. 89, 173505
- **LAVRIK, N. V. ; SEPANIAK, M. J. ; DATSKOS, P. G.** Cantilever transducers as a platform for chemical and biological sensors. *Rev. Sci. Instrum.,* 2004, vol. 75, 2229-2253
- **RAITERI, R. ; GRATTAROLA, M. ; BUTT, H. J. ; SKL'ADAL, P.** Micromechanical cantilever-based biosensors Sensors Actuators. *Sensors Actuators,* 2001, vol. B79, 115-126
- **NDIEYIRA, J. W. et al.** Surface-stress sensors for rapid and ultrasensitive detection of active free drugs in human serum. *Nat. Nanotechnol.,* 2014, vol. 9, 225-232
- **PATIL, S. B. et al.** Decoupling competing surface binding kinetics and reconfiguration of receptor footprint for ultrasensitive stress assays. *Nat. Nanotechnol.,* 2015, vol. 10, 899-907
- **MALVANKAR, N. S. ; YALCIN, S. E. ; TUOMINEN, M. T. ; LOVLEY, D. R.** Visualization of charge propagation along individual pili proteins using ambient electrostatic force microscopy. *Nat. Nanotechnol.,* 2014, vol. 9, 1012-1017
- **TETARD, L. ; PASSIAN, A. ; FARAHI, R. H. ; THUNDAT, T. ; DAVISON, B. H.** Opto-nanomechanical spectroscopic material characterization. *Nat. Nanotechnol.,* 2015, vol. 10, 870-877
- **DUPRES, V. et al.** Nanoscale mapping and functional analysis of individual adhesins on living bacteria. *Nat. Meth.,* 2005, vol. 2, 515-520

- **DUPRES, V.** Using nanotechniques to explore microbial surfaces. *Nat. Rev. Microbiol.,* 2004, vol. 2, 451-460
- **ZHANG, J. et al.** Rapid and label-free nanomechanical detection of biomarker transcripts in human RNA. *Nat. Nanotechnol.,* 2006, vol. 1, 214-220
- **BACKMANN, N. et al.** A label-free immunosensor array using single-chain antibody fragments. *Proc. Natl. Acad. Sci. USA,* 2005, vol. 102, 14587-14592
- **MUKHOPADHYAY, R. et al.** Cantilever sensor for nanomechanical detection of specific protein conformations. *Nano Lett.,* 2005, vol. 5, 2385-2388
- **GARCIA, R. ; KNOLL, A. W. ; RIEDO, E.** Advanced scanning probe lithography. *Nat. Nanotechnol.,* 2014, vol. 9, 577-587
- **LEE, K. B. ; PARK, S. J. ; MIRKIN, C. A. ; SMITH, J. C. ; MRKSICH, M.** Protein nanoarrays generated by dip-pen nanolithography. *Science,* 2002, vol. 295, 1702-1705
- **SALAITA, K. ; WANG, Y. ; MIRKIN, C. A.** Applications of dip-pen nanolithography. *Nat. Nanotechnol.,* 2007, vol. 2, 145-155
- **LOVE, J. C. ; ESTROFF, L. A. ; KRIEBEL, J. K. ; NUZZO, R. G. ; WHITESIDES, G. M.** Self-assembled monolayers of thiolates on metals as a Form of nanotechnology. *Chem. Rev.,* 2005, vol. 105, 1103-1169
- **CHO Y. R. ; ENTRESS R. M. ; WILLIAMS, D. H.** Synthesis of cell-wall analogues of vancomycin-resistant enterococci using solid phase peptide synthesis. *Tetrahedron Lett.,* 1997, vol. 38, 5229-5232
- **KAUFMAN, E. D. et al.** Probing protein adsorption onto mercaptoundecanoic acid stabilized gold nanoparticles and surfaces by quartz crystal microbalance and zeta-potential measurements. *Langmuir,* 2007, vol. 23, 6053-6062
- **WATARI, M. et al.** Investigating the molecular mechanisms of in-plane mechanochemistry on cantilever arrays. *J. Am. Chem. Soc.,* 2007, vol. 129, 601-607
- **STONEY, G. G.** The tension of metallic films deposited by electrolysis. *Proc. Roy. Soc. London, Ser. A.,* 1909, vol. 82, 172-175
- **JARVIS, W. R.** Epidemiology, appropriateness, and cost of vancomycin use. *Clin. Infect. Dis.,* 1998, vol. 26, 1200-1203
- **SIERADZKI, K. et al.** The development of vancomycin resistance in a patient with methicillin-resistant Staphylococcus aureus infection. *NEJM.,* 1999, vol. 340, 517-523
- **BURG, T. P. et al.** Weighing of biomolecules, single cells and single nanoparticles in fluid. *Nature,* 2007, vol. 446, 1066-1069
- **NIETO, M. ; PERKINS, H. R.** Modifications of the Acyl-D-alanyl-D-alanine terminus affecting complex-formation with vancomycin. *Biochem. J.,* 1971, vol. 123, 789-803
- **RODEGHIERO, F. ; CASTAMAN, G. ; DINI, E.** Epidemiological investigation of the prevalence of von Willebrand's disease. *Blood,* 1987, vol. 69, 454-459
- **AHMED, S. ; MATHEWS, A. S. ; BYEON, N. ; LAVASANIFAR, A. ; KAUR, K.** Peptide arrays for screening cancer specific peptides. *Anal. Chem.,* 2010, vol. 82, 7533-7541